# EUROPEAN PATENT APPLICATION

(11) **EP 2 273 266 A1**
(43) Date of publication of application: **12.01.2011**
(21) Application number: 09727199.3
(22) Date of filing: 30.03.2009
(51) Int. Cl.: G01N 33/50, A61K 39/395, A61K 45/00, A61P 35/00, C07K 14/325, C07K 14/47, C07K 16/18, G01N 33/15, G01N 33/53

(54) **PARASPORIN-1 RECEPTOR AND USE THEREOF**

(30) Priority: 31.03.2008 JP 2008090190
(71) Applicant: Fukuoka Prefectural Government, Fukuoka-shi Fukuoka 812-8577 (JP)
(72) Inventor: KATAYAMA, Hideki, Munakata-shi Fukuoka 811-3402 (JP); KUSAKA, Yoshitomo, Kurume-shi, Fukuoka 839-0814 (JP); MIZUKI, Eiichi, Kurume-shi Fukuoka 839-0861 (JP)
(74) Representative: Duckworth, Timothy John
(86) International application number: PCT/JP2009/056548
(87) International publication number: WO 2009/123145

(57) **Abstract**

The receptor of parasporin-1 has been found to be beclin-1. Based on the finding, the present invention provides a method of determining the sensitivity of a cell to parasporin-1, comprising measuring the expression level of beclin-1 polypeptide on the cell membrane or in a membrane fraction of a cell; an inhibitor of the cytotoxicity of parasporin-1, containing beclin-1 polypeptide etc.; a method of screening for a substance capable of inhibiting the cytotoxicity of parasporin-1, comprising evaluating the degree of binding of parasporin-1 to beclin-1 polypeptide in the presence of a test substance; a cell death inducer containing an antibody that specifically recognizes beclin-1 polypeptide; a method of screening for a substance capable of inducing cell death, comprising evaluating the binding of a test substance to beclin-1 polypeptide, and the like.

SEQUENCE LISTING

<110> Fukuoka Prefectural Government

<120> Parasporin-1 receptor and use thereof

<130> 091375

<150> JP 2008-090190
<151> 2008-03-31

<160> 6

<170> PatentIn version 3.2

<210> 1
<211> 2144
<212> DNA
<213> Homo sapiens


<220>
<221> CDS
<222> (134)..(1486)

<400> 1
gctgaagaca gagcgatggt agttctggag gcctcgctcc ggggccgacc cgaggccaca 60

gtgcctccgc ggtagaccgg acttgggtga cgggctccgg gctcccgagg tgaagagcat 120

cgggggctga ggg atg gaa ggg tct aag acg tcc aac aac agc acc atg 169
Met Glu Gly Ser Lys Thr Ser Asn Asn Ser Thr Met
1               5                   10

cag gtg agc ttc gtg tgc cag cgc tgc agc cag ccc ctg aaa ctg gac 217
Gln Val Ser Phe Val Cys Gln Arg Cys Ser Gln Pro Leu Lys Leu Asp
        15                  20                  25

acg agt ttc aag atc ctg gac cgt gtc acc atc cag gaa ctc aca gct 265
Thr Ser Phe Lys Ile Leu Asp Arg Val Thr Ile Gln Glu Leu Thr Ala
    30                  35                  40

cca tta ctt acc aca gcc cag gcg aaa cca gga gag acc cag gag gaa 313
Pro Leu Leu Thr Thr Ala Gln Ala Lys Pro Gly Glu Thr Gln Glu Glu
45                  50                  55                  60

gag act aac tca gga gag gag cca ttt att gaa act cct cgc cag gat 361
Glu Thr Asn Ser Gly Glu Glu Pro Phe Ile Glu Thr Pro Arg Gln Asp
                65                  70                  75

ggt gtc tct cgc aga ttc atc ccc cca gcc agg atg atg tcc aca gaa 409
Gly Val Ser Arg Arg Phe Ile Pro Pro Ala Arg Met Met Ser Thr Glu
            80                  85                  90

agt gcc aac agc ttc act ctg att ggg gag gca tct gat ggc ggc acc 457
Ser Ala Asn Ser Phe Thr Leu Ile Gly Glu Ala Ser Asp Gly Gly Thr
        95                  100                 105

atg gag aac ctc agc cga aga ctg aag gtc act ggg gac ctt ttt gac 505
Met Glu Asn Leu Ser Arg Arg Leu Lys Val Thr Gly Asp Leu Phe Asp
    110                 115                 120

atc atg tcg ggc cag aca gat gtg gat cac cca ctc tgt gag gaa tgc 553
Ile Met Ser Gly Gln Thr Asp Val Asp His Pro Leu Cys Glu Glu Cys
125                 130                 135                 140

aca gat act ctt tta gac cag ctg gac act cag ctc aac gtc act gaa 601
Thr Asp Thr Leu Leu Asp Gln Leu Asp Thr Gln Leu Asn Val Thr Glu
                145                 150                 155

aat gag tgt cag aac tac aaa cgc tgt ttg gag atc tta gag caa atg 649
Asn Glu Cys Gln Asn Tyr Lys Arg Cys Leu Glu Ile Leu Glu Gln Met
            160                 165                 170

aat gag gat gac agt gaa cag tta cag atg gag cta aag gag ctg gca 697

sn Glu Asp Asp Ser Glu Gln Leu Gln Met Glu Leu Lys Glu Leu Ala
175 180 185

cta gag gag gag agg ctg atc cag gag ctg gaa gac gtg gaa aag aac 745
Leu Glu Glu Glu Arg Leu Ile Gln Glu Leu Glu Asp Val Glu Lys Asn
190 195 200

cgc aag ata gtg gca gaa aat ctc gag aag gtc cag gct gag gct gag 793
Arg Lys Ile Val Ala Glu Asn Leu Glu Lys Val Gln Ala Glu Ala Glu
205 210 215 220

aga ctg gat cag gag gaa gct cag tat cag aga gaa tac agt gaa ttt 841
Arg Leu Asp Gln Glu Glu Ala Gln Tyr Gln Arg Glu Tyr Ser Glu Phe
225 230 235

aaa cga cag cag ctg gag ctg gat gat gag ctg aag agt gtt gaa aac 889
Lys Arg Gln Gln Leu Glu Leu Asp Asp Glu Leu Lys Ser Val Glu Asn
240 245 250

cag atg cgt tat gcc cag acg cag ctg gat aag ctg aag aaa acc aac 937
Gln Met Arg Tyr Ala Gln Thr Gln Leu Asp Lys Leu Lys Lys Thr Asn
255 260 265

gtc ttt aat gca acc ttc cac atc tgg cac agt gga cag ttt ggc aca 985
Val Phe Asn Ala Thr Phe His Ile Trp His Ser Gly Gln Phe Gly Thr
270 275 280

atc aat aac ttc agg ctg ggt cgc ctg ccc agt gtt ccc gtg gaa tgg 1033
Ile Asn Asn Phe Arg Leu Gly Arg Leu Pro Ser Val Pro Val Glu Trp
285 290 295 300

aat gag att aat gct gct tgg ggc cag act gtg ttg ctg ctc cat gct 1081
Asn Glu Ile Asn Ala Ala Trp Gly Gln Thr Val Leu Leu Leu His Ala
305 310 315

ctg gcc aat aag atg ggt ctg aaa ttt cag aga tac cga ctt gtt cct 1129
Leu Ala Asn Lys Met Gly Leu Lys Phe Gln Arg Tyr Arg Leu Val Pro
320 325 330

tac gga aac cat tca tat ctg gag tct ctg aca gac aaa tct aag gag 1177
Tyr Gly Asn His Ser Tyr Leu Glu Ser Leu Thr Asp Lys Ser Lys Glu
335 340 345

ctg ccg tta tac tgt tct ggg ggg ttg cgg ttt ttc tgg gac aac aag 1225
Leu Pro Leu Tyr Cys Ser Gly Gly Leu Arg Phe Phe Trp Asp Asn Lys
350 355 360

ttt gac cat gca atg gtg gct ttc ctg gac tgt gtg cag cag ttc aaa 1273
Phe Asp His Ala Met Val Ala Phe Leu Asp Cys Val Gln Gln Phe Lys
365 370 375 380

gaa gag gtt gag aaa ggc gag aca cgt ttt tgt ctt ccc tac agg atg 1321
Glu Glu Val Glu Lys Gly Glu Thr Arg Phe Cys Leu Pro Tyr Arg Met
385 390 395

gat gtg gag aaa ggc aag att gaa gac aca gga ggc agt ggc ggc tcc 1369
Asp Val Glu Lys Gly Lys Ile Glu Asp Thr Gly Gly Ser Gly Gly Ser
400 405 410

tat tcc atc aaa acc cag ttt aac tct gag gag cag tgg aca aaa gct 1417
Tyr Ser Ile Lys Thr Gln Phe Asn Ser Glu Glu Gln Trp Thr Lys Ala
415 420 425

ctc aag ttc atg ctg acg aat ctt aag tgg ggt ctt gct tgg gtg tcc 1465
Leu Lys Phe Met Leu Thr Asn Leu Lys Trp Gly Leu Ala Trp Val Ser
430 435 440

tca caa ttt tat aac aaa tga ctttttcct tagggggagg tttgccttaa 1516
Ser Gln Phe Tyr Asn Lys
445 450

aggcttttaa ttttgttttg tttgcaaaca tgttttaaat taaattcggg taatattaaa 1576

cagtacatgt ttacaatacc aaaaaagaaa aaatccacaa aagccacttt attttaaaat 1636

tcatgtgac agatactttc cagagctaca acatgccatc tatagttgcc agccctggtc 1696

agttttgatt cttaacccca tggactcctt tccctttctt ctctgaaaaa aactaattta 1756

aatttgcttt tctttttttt aactgagttg aattgagatt gatgtgtttt cactggattt 1816

ttatctctct caacttcctg cacttaacaa tatgaaatag aaacttttgt ctttactgag 1876

atgaggatat gtttgagatg cacagttgga taatgtggga aaatgacatc taagctttac 1936

ctggtcacca tgtgatgtga tcagatgctt gaaatttaac acttttcact tggttcttat 1996

actgaatgcc gactctgctc tgtgttagag atatgaaatg gtgtttgata ctgtttgaga 2056

cattatggag agatttaatt atttgtaata aaagatttgc tgcagtctga aaactgaaaa 2116

aaaaaaaaa aaaaaaaaa aaaaaaa 2144

<210> 2
<211> 450
<212> PRT
<213> Homo sapiens

<400> 2

Met Glu Gly Ser Lys Thr Ser Asn Asn Ser Thr Met Gln Val Ser Phe
1               5                   10                  15

Val Cys Gln Arg Cys Ser Gln Pro Leu Lys Leu Asp Thr Ser Phe Lys
            20                  25                  30

Ile Leu Asp Arg Val Thr Ile Gln Glu Leu Thr Ala Pro Leu Leu Thr
        35                  40                  45

Thr Ala Gln Ala Lys Pro Gly Glu Thr Gln Glu Glu Glu Thr Asn Ser
    50                  55                  60

Gly Glu Glu Pro Phe Ile Glu Thr Pro Arg Gln Asp Gly Val Ser Arg
65                  70                  75                  80

Arg Phe Ile Pro Pro Ala Arg Met Met Ser Thr Glu Ser Ala Asn Ser
                85                  90                  95

Phe Thr Leu Ile Gly Glu Ala Ser Asp Gly Gly Thr Met Glu Asn Leu
            100                 105                 110

Ser Arg Arg Leu Lys Val Thr Gly Asp Leu Phe Asp Ile Met Ser Gly
        115                 120                 125

Gln Thr Asp Val Asp His Pro Leu Cys Glu Glu Cys Thr Asp Thr Leu
    130                 135                 140

Leu Asp Gln Leu Asp Thr Gln Leu Asn Val Thr Glu Asn Glu Cys Gln
145                 150                 155                 160

Asn Tyr Lys Arg Cys Leu Glu Ile Leu Glu Gln Met Asn Glu Asp Asp
                165                 170                 175

Ser Glu Gln Leu Gln Met Glu Leu Lys Glu Leu Ala Leu Glu Glu Glu
            180                 185                 190

Arg Leu Ile Gln Glu Leu Glu Asp Val Glu Lys Asn Arg Lys Ile Val

195                 200                 205

Ala Glu Asn Leu Glu Lys Val Gln Ala Glu Ala Glu Arg Leu Asp Gln
    210                 215                 220

Glu Glu Ala Gln Tyr Gln Arg Glu Tyr Ser Glu Phe Lys Arg Gln Gln
225                 230                 235                 240

Leu Glu Leu Asp Asp Glu Leu Lys Ser Val Glu Asn Gln Met Arg Tyr
                245                 250                 255

Ala Gln Thr Gln Leu Asp Lys Leu Lys Lys Thr Asn Val Phe Asn Ala
            260                 265                 270

Thr Phe His Ile Trp His Ser Gly Gln Phe Gly Thr Ile Asn Asn Phe
        275                 280                 285

Arg Leu Gly Arg Leu Pro Ser Val Pro Val Glu Trp Asn Glu Ile Asn
    290                 295                 300

Ala Ala Trp Gly Gln Thr Val Leu Leu Leu His Ala Leu Ala Asn Lys
305                 310                 315                 320

Met Gly Leu Lys Phe Gln Arg Tyr Arg Leu Val Pro Tyr Gly Asn His
                325                 330                 335

Ser Tyr Leu Glu Ser Leu Thr Asp Lys Ser Lys Glu Leu Pro Leu Tyr
            340                 345                 350

Cys Ser Gly Gly Leu Arg Phe Phe Trp Asp Asn Lys Phe Asp His Ala
        355                 360                 365

Met Val Ala Phe Leu Asp Cys Val Gln Gln Phe Lys Glu Glu Val Glu
    370                 375                 380

Lys Gly Glu Thr Arg Phe Cys Leu Pro Tyr Arg Met Asp Val Glu Lys
385                 390                 395                 400

Gly Lys Ile Glu Asp Thr Gly Gly Ser Gly Gly Ser Tyr Ser Ile Lys
                405                 410                 415

Thr Gln Phe Asn Ser Glu Glu Gln Trp Thr Lys Ala Leu Lys Phe Met
            420                 425                 430

Leu Thr Asn Leu Lys Trp Gly Leu Ala Trp Val Ser Ser Gln Phe Tyr
        435                 440                 445

Asn Lys
    450

<210>  3
<211>  2031
<212>  DNA
<213>  Mus musculus

<220>
<221>  CDS

222>  (139)..(1485)

<400>  3
agtcttcagc tagccgaccg ggaagtagct gaagaccggg cgatgggaac tctggaggtc      60

tcgctccggg gcggacccga ggccaccgtg cccccgcggt agaacgagcc tgggtggcgg     120

gctccgggct cctgaggc atg gag ggg tct aag gcg tcc agc agc acc atg       171
                    Met Glu Gly Ser Lys Ala Ser Ser Ser Thr Met
                    1               5                   10

cag gtg agc ttc gtg tgc cag cgc tgt agc cag cct ctg aaa ctg gac       219
Gln Val Ser Phe Val Cys Gln Arg Cys Ser Gln Pro Leu Lys Leu Asp
            15                  20                  25

acg agc ttc aag atc ctg gac cgg gtc acc atc cag gaa ctc aca gct       267
Thr Ser Phe Lys Ile Leu Asp Arg Val Thr Ile Gln Glu Leu Thr Ala
        30                  35                  40

cca tta ctt acc aca gcc cag gcg aaa cca gga gag acc cag gag gaa       315
Pro Leu Leu Thr Thr Ala Gln Ala Lys Pro Gly Glu Thr Gln Glu Glu
    45                  50                  55

gag gct aac tca gga gag gag cca ttt att gaa act cgc cag gat ggt       363
Glu Ala Asn Ser Gly Glu Glu Pro Phe Ile Glu Thr Arg Gln Asp Gly
60                  65                  70                  75

gtc tct cga aga ttc atc ccc cca gcc agg atg atg tct aca gaa agt       411
Val Ser Arg Arg Phe Ile Pro Pro Ala Arg Met Met Ser Thr Glu Ser
                80                  85                  90

gct aat agc ttc act ctg atc ggg gag gca tct gat ggt ggc acc atg       459
Ala Asn Ser Phe Thr Leu Ile Gly Glu Ala Ser Asp Gly Gly Thr Met
            95                  100                 105

gag aac ctc agc cgg aga ctc aag gtc act gga gac ctg ttt gac atc       507
Glu Asn Leu Ser Arg Arg Leu Lys Val Thr Gly Asp Leu Phe Asp Ile
        110                 115                 120

atg tcg ggc cag acc gat gtg gat cac ccg ctg tgt gag gaa tgc aca       555
Met Ser Gly Gln Thr Asp Val Asp His Pro Leu Cys Glu Glu Cys Thr
    125                 130                 135

gac act ctt tta gac cag ctg gac act cag ctc aat gtc act gag aat       603
Asp Thr Leu Leu Asp Gln Leu Asp Thr Gln Leu Asn Val Thr Glu Asn
140                 145                 150                 155

gaa tgt cag aac tac aaa cgc tgt ttg gag atc cta gag cag atg aat       651
Glu Cys Gln Asn Tyr Lys Arg Cys Leu Glu Ile Leu Glu Gln Met Asn
                160                 165                 170

gag gat gac agt gag cag ctg cag agg gag ctg aag gag ctg gcc ttg       699
Glu Asp Asp Ser Glu Gln Leu Gln Arg Glu Leu Lys Glu Leu Ala Leu
            175                 180                 185

gag gag gag agg ctg atc cag gag ctg gaa gat gtg gaa aag aac cgc       747
Glu Glu Glu Arg Leu Ile Gln Glu Leu Glu Asp Val Glu Lys Asn Arg
        190                 195                 200

aag gtg gtg gca gag aac ctg gag aag gtc cag gct gag gcg gag aga       795
Lys Val Val Ala Glu Asn Leu Glu Lys Val Gln Ala Glu Ala Glu Arg
    205                 210                 215

ttg gac cag gag gaa gct cag tac cag cgg gag tat agt gag ttt aaa       843
Leu Asp Gln Glu Glu Ala Gln Tyr Gln Arg Glu Tyr Ser Glu Phe Lys
220                 225                 230                 235

agg cag cag ctg gag ttg gat gac gaa ctc aag agt gtg gag aac cag       891
Arg Gln Gln Leu Glu Leu Asp Asp Glu Leu Lys Ser Val Glu Asn Gln
                240                 245                 250

gtg cgc tac gcc cag atc cag ctg gac aag ctc aag aaa acc aat gtc       939
Val Arg Tyr Ala Gln Ile Gln Leu Asp Lys Leu Lys Lys Thr Asn Val
            255                 260                 265

ttc aat gcc acc ttc cac atc tgg cac agc gga cag ttt ggc aca atc     987
Phe Asn Ala Thr Phe His Ile Trp His Ser Gly Gln Phe Gly Thr Ile
        270                 275                 280

aat aat ttc aga ctg ggt cgc ttg ccc agt gtt cct gtg gag tgg aat     1035
Asn Asn Phe Arg Leu Gly Arg Leu Pro Ser Val Pro Val Glu Trp Asn
    285                 290                 295

gaa atc aat gct gcc tgg ggc cag aca gtg ctg ctg ctc cat gct ttg     1083
Glu Ile Asn Ala Ala Trp Gly Gln Thr Val Leu Leu Leu His Ala Leu
300                 305                 310                 315

gcc aat aag atg ggt ctg aag ttt cag agg tac cga ctt gtt ccc tat     1131
Ala Asn Lys Met Gly Leu Lys Phe Gln Arg Tyr Arg Leu Val Pro Tyr
                320                 325                 330

gga aat cat tcc tat ctg gag tct ctg aca gac aaa tct aag gag ttg     1179
Gly Asn His Ser Tyr Leu Glu Ser Leu Thr Asp Lys Ser Lys Glu Leu
            335                 340                 345

ccg tta tac tgt tct ggg ggt ttg cgg ttt ttc tgg gac aac aag ttt     1227
Pro Leu Tyr Cys Ser Gly Gly Leu Arg Phe Phe Trp Asp Asn Lys Phe
        350                 355                 360

gac cat gca atg gta gct ttt ctg gac tgt gtg cag cag ttc aaa gaa     1275
Asp His Ala Met Val Ala Phe Leu Asp Cys Val Gln Gln Phe Lys Glu
    365                 370                 375

gag gtg gaa aaa gga gag act cga ttt tgt ctt ccg tac agg atg gac     1323
Glu Val Glu Lys Gly Glu Thr Arg Phe Cys Leu Pro Tyr Arg Met Asp
380                 385                 390                 395

gtg gag aaa ggc aag att gaa gac act gga ggc agt ggc ggc tcc tat     1371
Val Glu Lys Gly Lys Ile Glu Asp Thr Gly Gly Ser Gly Gly Ser Tyr
                400                 405                 410

tcc atc aaa acc cag ttt aac tcg gag gag cag tgg aca aaa gcg ctc     1419
Ser Ile Lys Thr Gln Phe Asn Ser Glu Glu Gln Trp Thr Lys Ala Leu
            415                 420                 425

aag ttc atg ctg acc aat ctc aag tgg ggt ctt gcc tgg gtg tcc tca     1467
Lys Phe Met Leu Thr Asn Leu Lys Trp Gly Leu Ala Trp Val Ser Ser
        430                 435                 440

cag ttc tat aac aag tga cttgctcctt aggggatgtt tgcctttaag            1515
Gln Phe Tyr Asn Lys
    445

gttttatact ttgtttggtt tggaaagatg ctttaaatta aatttgggta atattaaacc   1575

acatgtttac aataccaaaa tccacaaaag ctactttatt ttcaaatatg acagatagtt   1635

tccagagtac gccatgtata gcaaagaacc ctgccatagt tttgactcag ccccatgcat   1695

cctttccctc tttcctgaaa acaactaatt taaatttgct ttgttttctt ttttaagttg   1755

aattgacgtt aatgtgtttt cactggattt tatctctctc aacttcctgc acttaaaatt   1815

tgaaacagca aaggtttgag atgagatgct tgtggcacac agttgggtga tgtggggaaa   1875

ggacaccggg tcaggagttg caagtttaac tccgtcctca cttgtagcat tgaatgcctc   1935

ctgtgctgtc tagtgggact acagaatgct gtttgatact gtgtgcgacg tggagagatt   1995

taattatttg taataaagga tttgctatgg tctatt                             2031

<210>  4
<211>  448
<212>  PRT
<213>  Mus musculus

<400>  4

Met Glu Gly Ser Lys Ala Ser Ser Ser Thr Met Gln Val Ser Phe Val

                5                   10                  15

Cys Gln Arg Cys Ser Gln Pro Leu Lys Leu Asp Thr Ser Phe Lys Ile
            20                  25                  30

Leu Asp Arg Val Thr Ile Gln Glu Leu Thr Ala Pro Leu Leu Thr Thr
        35                  40                  45

Ala Gln Ala Lys Pro Gly Glu Thr Gln Glu Glu Glu Ala Asn Ser Gly
    50                  55                  60

Glu Glu Pro Phe Ile Glu Thr Arg Gln Asp Gly Val Ser Arg Arg Phe
65                  70                  75                  80

Ile Pro Pro Ala Arg Met Met Ser Thr Glu Ser Ala Asn Ser Phe Thr
                85                  90                  95

Leu Ile Gly Glu Ala Ser Asp Gly Gly Thr Met Glu Asn Leu Ser Arg
            100                 105                 110

Arg Leu Lys Val Thr Gly Asp Leu Phe Asp Ile Met Ser Gly Gln Thr
        115                 120                 125

Asp Val Asp His Pro Leu Cys Glu Glu Cys Thr Asp Thr Leu Leu Asp
    130                 135                 140

Gln Leu Asp Thr Gln Leu Asn Val Thr Glu Asn Glu Cys Gln Asn Tyr
145                 150                 155                 160

Lys Arg Cys Leu Glu Ile Leu Glu Gln Met Asn Glu Asp Asp Ser Glu
                165                 170                 175

Gln Leu Gln Arg Glu Leu Lys Glu Leu Ala Leu Glu Glu Glu Arg Leu
            180                 185                 190

Ile Gln Glu Leu Glu Asp Val Glu Lys Asn Arg Lys Val Val Ala Glu
        195                 200                 205

Asn Leu Glu Lys Val Gln Ala Glu Ala Glu Arg Leu Asp Gln Glu Glu
    210                 215                 220

Ala Gln Tyr Gln Arg Glu Tyr Ser Glu Phe Lys Arg Gln Gln Leu Glu
225                 230                 235                 240

Leu Asp Asp Glu Leu Lys Ser Val Glu Asn Gln Val Arg Tyr Ala Gln
                245                 250                 255

Ile Gln Leu Asp Lys Leu Lys Lys Thr Asn Val Phe Asn Ala Thr Phe
            260                 265                 270

His Ile Trp His Ser Gly Gln Phe Gly Thr Ile Asn Asn Phe Arg Leu
        275                 280                 285

Gly Arg Leu Pro Ser Val Pro Val Glu Trp Asn Glu Ile Asn Ala Ala
    290                 295                 300

Trp Gly Gln Thr Val Leu Leu Leu His Ala Leu Ala Asn Lys Met Gly
305                 310                 315                 320

Leu Lys Phe Gln Arg Tyr Arg Leu Val Pro Tyr Gly Asn His Ser Tyr
                325                 330                 335

Leu Glu Ser Leu Thr Asp Lys Ser Lys Glu Leu Pro Leu Tyr Cys Ser
            340                 345                 350

Gly Gly Leu Arg Phe Phe Trp Asp Asn Lys Phe Asp His Ala Met Val
        355                 360                 365

Ala Phe Leu Asp Cys Val Gln Gln Phe Lys Glu Glu Val Glu Lys Gly
    370                 375                 380

Glu Thr Arg Phe Cys Leu Pro Tyr Arg Met Asp Val Glu Lys Gly Lys
385                 390                 395                 400

Ile Glu Asp Thr Gly Gly Ser Gly Gly Ser Tyr Ser Ile Lys Thr Gln
                405                 410                 415

Phe Asn Ser Glu Glu Gln Trp Thr Lys Ala Leu Lys Phe Met Leu Thr
            420                 425                 430

Asn Leu Lys Trp Gly Leu Ala Trp Val Ser Ser Gln Phe Tyr Asn Lys
        435                 440                 445

<210>  5
<211>  2140
<212>  DNA
<213>  Rattus norvegicus

<220>
<221>  CDS
<222>  (225)..(1571)

<400>  5
   tggatctgga ccaggggtcc ttgcgtcagc tctcgtcaag gcgtcacttc tgggcccggg      60
   cggaagtctt cagctagccg accgggaagt agctgaagac cgggcgatgg gaactctgga     120
   ggtctcgctc tggggcggac ccgaggccac cgttcccccg cggtagagcg agcctaggtg     180
   gcgggctccg gggtcctgag gtgaagagcg tcggggccta aaga atg gag ggg tct     236
                                                    Met Glu Gly Ser
                                                    1
   aag gcg tcc agc agc acc atg cag gtg agc ttc gtg tgc cag cgc tgt      284
   Lys Ala Ser Ser Ser Thr Met Gln Val Ser Phe Val Cys Gln Arg Cys
   5                   10                  15                  20
   agc cag cct ctg aaa ctg gac acg agc ttc aag atc ctg gac cga gtg      332
   Ser Gln Pro Leu Lys Leu Asp Thr Ser Phe Lys Ile Leu Asp Arg Val
                   25                  30                  35
   acc att cag gaa ctc aca gct cca tta ctt acc aca gcc cag gcg aaa      380
   Thr Ile Gln Glu Leu Thr Ala Pro Leu Leu Thr Thr Ala Gln Ala Lys
               40                  45                  50
   cca gga gag agc cag gag gaa gag gct aac tca gga gag gag cca ttt      428
   Pro Gly Glu Ser Gln Glu Glu Glu Ala Asn Ser Gly Glu Glu Pro Phe
           55                  60                  65
   att gaa act cgc cag gat ggt gtc tct cga aga ttc atc ccc cca gcc      476
   Ile Glu Thr Arg Gln Asp Gly Val Ser Arg Arg Phe Ile Pro Pro Ala
   70                    75                    80
   agg atg atg tct aca gaa agt gct aat agc ttc act ctg atc ggg gag          524
   Arg Met Met Ser Thr Glu Ser Ala Asn Ser Phe Thr Leu Ile Gly Glu
   85                  90                  95                  100
   gca tct gat ggt ggc acc atg gag aac ctc agc cgg aga ctc aag gtc          572
   Ala Ser Asp Gly Gly Thr Met Glu Asn Leu Ser Arg Arg Leu Lys Val
                   105                 110                 115
   act gga gac ctt ttt gac atc atg tct ggc cag aca gat gtg gat cac          620
   Thr Gly Asp Leu Phe Asp Ile Met Ser Gly Gln Thr Asp Val Asp His
               120                 125                 130
   cca ctg tgt gag gaa tgc aca gat act ctt tta gac cag ctg gac act          668
   Pro Leu Cys Glu Glu Cys Thr Asp Thr Leu Leu Asp Gln Leu Asp Thr
           135                 140                 145
   cag ctc aat gtt act gaa aac gag tgt cag aac tac aaa cgc tgt ttg          716
   Gln Leu Asn Val Thr Glu Asn Glu Cys Gln Asn Tyr Lys Arg Cys Leu
       150                 155                 160
   gag atg ttg gag caa atg aat gag ggc gac agt gaa cag cta cag agg          764
   Glu Met Leu Glu Gln Met Asn Glu Gly Asp Ser Glu Gln Leu Gln Arg
   165                 170                 175                 180
   gag ctg aag gag ttg gcc ttg gag gag gag agg ctg atc cag gag ctg          812
   Glu Leu Lys Glu Leu Ala Leu Glu Glu Glu Arg Leu Ile Gln Glu Leu
                   185                 190                 195
   gaa gat gtg gaa aaa aac cga aag gtg gtg gca gaa aac ctg gag aag          860
   Glu Asp Val Glu Lys Asn Arg Lys Val Val Ala Glu Asn Leu Glu Lys
               200                 205                 210
   gtc cag gct gag gcg gag aga ctg gac cag gag gaa gct cag tac cag          908
   Val Gln Ala Glu Ala Glu Arg Leu Asp Gln Glu Glu Ala Gln Tyr Gln
           215                 220                 225
   cga gaa tat agt gaa ttt aaa agg cag cag ctg gag ctg gat gat gag          956
   Arg Glu Tyr Ser Glu Phe Lys Arg Gln Gln Leu Glu Leu Asp Asp Glu
       230                 235                 240
   ctc aag agt gta gag aac cag atg cgc tat gcc cag atg cag ctg gac          1004
   Leu Lys Ser Val Glu Asn Gln Met Arg Tyr Ala Gln Met Gln Leu Asp
   245                 250                 255                 260
   aag ctc aag aaa acc aat gtc ttc aat gcg acc ttc cat atc tgg cac          1052
   Lys Leu Lys Lys Thr Asn Val Phe Asn Ala Thr Phe His Ile Trp His
                   265                 270                 275
   agc gga caa ttt ggc acg atc aat aat ttc aga ctg ggt cgc ttg ccc          1100
   Ser Gly Gln Phe Gly Thr Ile Asn Asn Phe Arg Leu Gly Arg Leu Pro
               280                 285                 290
   agt gct cct gtg gaa tgg aat gaa atc aat gct gcc tgg ggc cag aca          1148
   Ser Ala Pro Val Glu Trp Asn Glu Ile Asn Ala Ala Trp Gly Gln Thr
           295                 300                 305
   gtg ttg ttg ctc cat gct ttg gcc aat aag atg ggt ctg aaa ttt cag          1196
   Val Leu Leu Leu His Ala Leu Ala Asn Lys Met Gly Leu Lys Phe Gln
       310                 315                 320
   agg tac cga ctt gtt ccc tat gga aat cac tcg tat ctg gag tcc ctg          1244
   Arg Tyr Arg Leu Val Pro Tyr Gly Asn His Ser Tyr Leu Glu Ser Leu
   325                 330                 335                 340
   aca gac aaa tct aag gag ttg ccg ttg tac tgt tct ggg ggt ttg cgg          1292
   Thr Asp Lys Ser Lys Glu Leu Pro Leu Tyr Cys Ser Gly Gly Leu Arg
                   345                 350                 355
   ttt ttc tgg gac aac aag ttt gac cat gca atg gta gct ttt ctg gac          1340
   Phe Phe Trp Asp Asn Lys Phe Asp His Ala Met Val Ala Phe Leu Asp
               360                 365                 370
   tgt gtg cag cag ttc aaa gaa gag gtg gaa aaa gga gag act cga ttt          1388
   Cys Val Gln Gln Phe Lys Glu Glu Val Glu Lys Gly Glu Thr Arg Phe
           375                 380                 385
   tgt ctt ccg tac agg atg gac gtg gag aaa ggc aag att gaa gac act      1436
   Cys Leu Pro Tyr Arg Met Asp Val Glu Lys Gly Lys Ile Glu Asp Thr
       390                 395                 400
   gga ggc agt ggc ggc tcc tat tcc atc aaa acc cag ttt aac tct gag      1484
   Gly Gly Ser Gly Gly Ser Tyr Ser Ile Lys Thr Gln Phe Asn Ser Glu
   405                 410                 415                 420
   gag cag tgg aca aag gcg ctc aag ttc atg ctg acg aat ctc aag tgg      1532
   Glu Gln Trp Thr Lys Ala Leu Lys Phe Met Leu Thr Asn Leu Lys Trp
                   425                 430                 435
   ggt ctt gct tgg gtg tcc tca cag ttc tat aac aag tga cttgctcctt      1581
   Gly Leu Ala Trp Val Ser Ser Gln Phe Tyr Asn Lys
               440                 445
   aggggatatt tgcctttaag gttttacatt ttgtttggtt tggaaagatg ctttaaatta      1641
   aatttgggta atattaaacc acatgtttac aataccagaa tccacaaaag ctactttatt      1701
   ttacaatatg acagttttca gagcacagca cgccatgtat agcaaagagc cctgccgtag      1761
   tttggctcaa ccccatgctg tcctttccct cttccctgga aacaactaat ttaaatttgc      1821
   tttgttttct tttttaagtt gaattgacat taatgtgttt tcactggatt ttatctctct      1881
   caacttcctg tacttgaaac agaaaagttt tgagatgaga tgcttgtggc acacagttgg      1941
   gtaatgtggg gaagggacaa gaggtaaagg tttaactctg tcctcacttc tagtattgaa      2001
   cgcttactgt gctaactatt ggaattacaa aatgctgttt gatactgttt gagatgtgga      2061
   aggatttaat tatttgtaat aaaggatttg ctatggtcta ttaattaaaa aaaaaaaaaa      2121
   aaaaaaaaaa aaaaaaaaa                                                   2140

<210>  6
<211>  448
<212>  PRT
<213>  Rattus norvegicus

<400>  6

Met Glu Gly Ser Lys Ala Ser Ser Ser Thr Met Gln Val Ser Phe Val
1               5                   10                  15

Cys Gln Arg Cys Ser Gln Pro Leu Lys Leu Asp Thr Ser Phe Lys Ile
            20                  25                  30

Leu Asp Arg Val Thr Ile Gln Glu Leu Thr Ala Pro Leu Leu Thr Thr
        35                  40                  45

Ala Gln Ala Lys Pro Gly Glu Ser Gln Glu Glu Glu Ala Asn Ser Gly
    50                  55                  60

Glu Glu Pro Phe Ile Glu Thr Arg Gln Asp Gly Val Ser Arg Arg Phe
65                  70                  75                  80

Ile Pro Pro Ala Arg Met Met Ser Thr Glu Ser Ala Asn Ser Phe Thr
                85                  90                  95

Leu Ile Gly Glu Ala Ser Asp Gly Gly Thr Met Glu Asn Leu Ser Arg
            100                 105                 110

Arg Leu Lys Val Thr Gly Asp Leu Phe Asp Ile Met Ser Gly Gln Thr
        115                 120                 125

Asp Val Asp His Pro Leu Cys Glu Glu Cys Thr Asp Thr Leu Leu Asp
    130                 135                 140

Gln Leu Asp Thr Gln Leu Asn Val Thr Glu Asn Glu Cys Gln Asn Tyr
145                 150                 155                 160

Lys Arg Cys Leu Glu Met Leu Glu Gln Met Asn Glu Gly Asp Ser Glu
                165                 170                 175

Gln Leu Gln Arg Glu Leu Lys Glu Leu Ala Leu Glu Glu Glu Arg Leu
            180                 185                 190

Ile Gln Glu Leu Glu Asp Val Glu Lys Asn Arg Lys Val Val Ala Glu
        195                 200                 205

Asn Leu Glu Lys Val Gln Ala Glu Ala Glu Arg Leu Asp Gln Glu Glu
    210                 215                 220

Ala Gln Tyr Gln Arg Glu Tyr Ser Glu Phe Lys Arg Gln Gln Leu Glu
225                 230                 235                 240

Leu Asp Asp Glu Leu Lys Ser Val Glu Asn Gln Met Arg Tyr Ala Gln
                245                 250                 255

Met Gln Leu Asp Lys Leu Lys Lys Thr Asn Val Phe Asn Ala Thr Phe
            260                 265                 270

His Ile Trp His Ser Gly Gln Phe Gly Thr Ile Asn Asn Phe Arg Leu
        275                 280                 285

Gly Arg Leu Pro Ser Ala Pro Val Glu Trp Asn Glu Ile Asn Ala Ala
    290                 295                 300

Trp Gly Gln Thr Val Leu Leu Leu His Ala Leu Ala Asn Lys Met Gly
305                 310                 315                 320

Leu Lys Phe Gln Arg Tyr Arg Leu Val Pro Tyr Gly Asn His Ser Tyr
                325                 330                 335

Leu Glu Ser Leu Thr Asp Lys Ser Lys Glu Leu Pro Leu Tyr Cys Ser
            340                 345                 350

Gly Gly Leu Arg Phe Phe Trp Asp Asn Lys Phe Asp His Ala Met Val
        355                 360                 365

Ala Phe Leu Asp Cys Val Gln Gln Phe Lys Glu Glu Val Glu Lys Gly
    370                 375                 380

Glu Thr Arg Phe Cys Leu Pro Tyr Arg Met Asp Val Glu Lys Gly Lys
385                 390                 395                 400

Ile Glu Asp Thr Gly Gly Ser Gly Gly Ser Tyr Ser Ile Lys Thr Gln
                405                 410                 415

Phe Asn Ser Glu Glu Gln Trp Thr Lys Ala Leu Lys Phe Met Leu Thr
            420                 425                 430

Asn Leu Lys Trp Gly Leu Ala Trp Val Ser Ser Gln Phe Tyr Asn Lys
        435                 440                 445

## Description

### Technical Field

The present invention relates to a use of beclin-1 as a receptor of parasporin-1. More specifically, the present invention relates to a method and agent for determining the sensitivity of cells to parasporin-1 using beclin-1 or an antibody against beclin-1, a method and agent for inhibiting the cytotoxicity of parasporin-1, a method of screening for a cell death inducer, an antitumor agent or a substance capable of inducing cell death, and the like.

### Background Art

Bacillus thuringiensis produces a proteinous inclusion body in the sporulation stage. This inclusion body is toxic to the larvae of certain insects (Non-patent Document 1). The present inventors discovered a toxin protein that is toxic to mammalian cells in large screening of inclusion bodies of B. thuringiensis (Non-patent Document 2). The biochemical properties of these proteins, including cell specificity and cytotoxicity, are very diverse; that is, some toxin proteins are toxic to a broad range of cell lines, and other proteins are toxic to a very limited range of cell lines (Non-patent Documents 2 to 5). These proteins differed from the Cyt protein of B. thuringiensis, which is toxic to mammalian cells and possesses hemolytic activity, because these proteins did not exhibit hemolytic activity.

Parasporin proteins can roughly be classified into four groups according to primary structure similarity, and 13 kinds of parasporin proteins have been identified so far (see the website http://parasporin.fitc.pref.fukuoka.jp/). The individual parasporin proteins have totally different specificities to cells and molecular weights.

Parasporin-1 is produced as an 81-kDa toxin precursor by the B. thuringiensis A1190 strain, and exhibits cytotoxicity upon partial degradation by treatment with a protease such as trypsin. The active form parasporin-1 is a complex consisting of a 56-kDa protein and a 15-kDa protein, exhibiting specific cytotoxic activity (Non-patent Document 14). HeLa, which is a human cervical carcinoma cell line, and HL-60, which is a human promyelocytic leukemia cell line, are highly sensitive to parasporin-1, whereas normal human uterine smooth muscle cells and Caco-2, which is a colorectal cancer cell line, are little sensitive. So far, in a test of the sensitivity of 15 kinds of cell lines, including normal cell lines, to parasporin, sensitivity was noted in 4 cell lines. This demonstrates that parasporin-1 is a cytotoxin possessing extremely high cell specificity (Non-patent Document 14). Parasporin-1, in its toxicity induction process, induces an elevation of the concentration of cytoplasmic free Ca²⁺ accompanying a Ca²⁺ influx, which triggers cell death. Since this was suppressed by suramin, which is an inhibitor of G-protein, it has been estimated that the Ca²⁺ influx induced by parasporin-1 is mediated by an intracellular signal transduction system via G-protein (Non-patent Document 6). Furthermore, in parasporin-1-treated HeLa cells, not only degradation of Poly(ADP-ribose) polymerase and Caspase-3 activation (Non-patent Document 6), but also degradation of DNA into nucleosome units and translocation of phosphatidylserine are noted; therefore, parasporin-1 is a toxin that induces apoptosis to target cells.

Because parasporin-1 is toxic to a very limited range of cells and induces apoptosis to targeted cells, its applications to the fields of pharmaceuticals, cell identifying reagents, and cell biology are expectable by understanding the action mechanisms of parasporin-1.
Particularly, because the binding of the toxin to a receptor is the most fundamental reaction in the toxic action, its identification is thought to be important. However, the receptor of parasporin-1 has not yet been identified.

Meanwhile, beclin-1 is a publicly known protein first identified as an autophagy-related protein (Non-patent Document 7), and has been reported to act as a cancer suppressor (Non-patent Documents 8 to 10). Furthermore, beclin-1 has been reported to be an intracellular protein that interacts with Bcl-2 and Bcl-X_{L}, which are factors that control apoptosis (Non-patent Documents 11 to 13). However, it is not known at all that beclin-1 protein is capable of being exposed to cell surfaces, and that beclin-1 is capable of functioning as a receptor.
non-patent document 1: Schnepf, E. et al., Microbiol. Mol. Biol. Rev. 62, 775-806 (1998)
non-patent document 2: Mizuki, E. et al., J. Appl. Microbiol. 86, 477-486 (1999)
non-patent document 3: Ito, A. et al., J. Biol. Chem. 279, 21282-21286 (2004)
non-patent document 4: Lee, D. W. et al., Biochem. Biophys. Res. Commun. 272, 218-223 (2000)
non-patent document 5: Yamashita, S. et al., Can J. Microbiol. 46, 913-919 (2000)
non-patent document 6: Katayama, H. et al., J. Biol. Chem. 282, 7742-7752 (2007)
non-patent document 7: Liang, H. X. et al., Nature 402, 672-676 (1999)
non-patent document 8: Atia, V. M. et al., Genomics 59, 59-65 (1999)
non-patent document 9: Qu, X. et al., J. Clin. Investig. 112, 1809-1820 (2003)
non-patent document 10: Yue, Z. et al., Proc. Natl. Acad. U. S. A., b, 15077-15082(2003)
non-patent document 11: Pattingre, S. et al., Cell, 122, 927-939 (2005)
non-patent document 12: Liang, X. H. et al., J. Virol. 72, 8586-8596 (1998)
non-patent document 13: Furuya, D. et al., Exp. Cell Res. 307, 26-40 (2005)
non-patent document 14: Katayama, H. et al., J. Biochem. 137, 17-25 (2005)

### Disclosure of the Invention

### Problems to Be Solved by the Invention

In view of the above-described circumstances, the present invention aims to identify the parasporin-1 receptor on a target cell, which is thought to be most important in the induction of the cytotoxicity of parasporin-1, and provide a new antitumor drug that targets the receptor, a screening method therefor, a tool for cell sorting and the like.

### Means of Solving the Problems

The present inventors, in order to explain the cell specificity of parasporin-1, searched for, and identified, the receptor of parasporin-1 using a photo-reactive chemical cross linker. Several candidate proteins for the receptor of parasporin-1 were separated by two-dimensional electrophoresis, and analyzed by the mass finger printing method utilizing a mass spectrometer; as a result, it was found that the parasporin-1-binding protein is beclin-1. An analysis using an anti-beclin-1 antibody and flow cytometry showed that beclin-1, which had been thought to be an intracellular protein, was localized outside of the cell membrane, and the binding of parasporin-1 to HeLa cells was inhibited by the anti-beclin-1 antibody. Furthermore, when the expression level of beclin-1 exposed on cell surfaces by the anti-beclin-1 antibody was examined, the expression level was high in HeLa cells, which are parasporin-1-sensitive cells, and the expression level was extremely low in Caco-2 and normal uterine smooth muscle cells, which are non-parasporin-1-sensitive cells. The anti-beclin-1 antibody (anti-peptide antibody) suppressed the binding of parasporin-1 to cell surfaces, and inhibited the cytotoxic effect of parasporin-1. Meanwhile, the anti-beclin-1 antibody (as a whole) exhibited cytotoxic activity by itself, suggesting that in the cell death, caspase-8 activation may be involved as with parasporin-1. In the results above, the present inventors found that the receptor of parasporin-1 is beclin-1, and that beclin-1 exposed on cell surfaces is one factor to determine the parasporin-1 sensitivity of cells, and have completed the present invention described below.

Accordingly, the present invention related to the following:
[1] A method of determining the sensitivity of a cell to parasporin-1, comprising measuring the expression level of beclin-1 polypeptide on the cell membrane or in a membrane fraction of a cell, and determining the sensitivity of the cell to parasporin-1 on the basis of a positive correlation between the expression level of beclin-1 polypeptide and the sensitivity to parasporin-1.
[2] The method described in [1], wherein the measurement of the expression level of beclin-1 polypeptide is performed using an antibody that specifically recognizes beclin-1 polypeptide.
[3] An agent for determining the sensitivity of a cell to parasporin-1, comprising an antibody that specifically recognizes beclin-1 polypeptide.
[4] An inhibitor of the cytotoxicity of parasporin-1, comprising beclin-1 polypeptide or an antibody that specifically recognizes beclin-1 polypeptide.
[5] A method of inhibiting the cytotoxicity of parasporin-1, comprising administering beclin-1 polypeptide or an antibody that specifically recognizes beclin-1 polypeptide.
[6] A method of screening for a substance capable of inhibiting the cytotoxicity of parasporin-1, comprising the steps shown below:
   (I) evaluating the degree of binding of parasporin-1 to beclin-1 polypeptide in the presence of a test substance, and
   (II) selecting a substance that has inhibited the binding of parasporin-1 to beclin-1 polypeptide as a substance capable of inhibiting the cytotoxicity of parasporin-1.
[7] A combination comprising parasporin-1 and beclin-1 polypeptide.
[8] A cell death inducer comprising an antibody that specifically recognizes beclin-1 polypeptide.
[9] An antitumor agent comprising an antibody that specifically recognizes beclin-1 polypeptide.
[10] A method of inducing cell death, comprising bringing an antibody that specifically recognizes beclin-1 polypeptide into contact with a cell.
[11] The method described in [10], wherein the cell is a tumor cell.
[12] A method of screening for a substance capable of inducing cell death, comprising the steps shown below:
   (I) evaluating the degree of binding of a test substance to beclin-1 polypeptide, and
   (II) selecting a test substance that has bound to beclin-1 polypeptide as a substance capable of inducing cell death.
[13] The method described in [12], wherein the beclin-1 polypeptide used in (I) is beclin-1 polypeptide expressed on the cell membrane.
[14] An antibody that specifically recognizes beclin-1 polypeptide, for use in inducing cell death.
[15] An antibody that specifically recognizes beclin-1 polypeptide, for use in preventing or treating a tumor.
[16] A method of inducing cell death in a mammal, comprising administering to the mammal an effective amount of an antibody that specifically recognizes beclin-1 polypeptide.
[17] A method of preventing or treating a tumor in a mammal, comprising administering to the mammal an effective amount of an antibody that specifically recognizes beclin-1 polypeptide.

### Effect of the Invention

Provided by the present invention are various uses of beclin-1 as a receptor of parasporin-1.
Using the method of the present invention for determining sensitivity to parasporin-1, it is possible to objectively determine the sensitivity of various cells to parasporin-1 on the basis of the expression level of parasporin-1 receptor definitely supported by molecular biologically.
Using the inhibitor of the cytotoxicity of parasporin-1 of the present invention, it is possible to potently suppress the cell death (apoptosis) induced by parasporin-1.
Using the cell death inducer of the present invention, it is possible to induce cell death (apoptosis) specifically to cells expressing beclin-1 polypeptide on the cell membrane or in a membrane fraction, and the inducer is useful as a cell-specific antitumor agent.

### Brief Description of the Drawings

Fig. 1 shows the binding of parasporin-1 labeled with Alexa Fluor 488 dye to HeLa cells. (A) Observations using a light microscope (A-C) and a fluorescence microscope (D-F). (B) Observations using a flow cytometer.
Fig. 2 shows correlations between parasporin-1 sensitivity and parasporin-1 affinity. (A) Parasporin-1 susceptibilities of various cell lines. (B) Affinity of parasporin-1 to cell surfaces of various cell lines.
Fig. 3 shows screening for and identification of parasporin-1 receptor using SBED-labeled parasporin-1. (A) Detection of parasporin-1-binding protein by Western blotting (one-dimensional electrophoresis). (B and C) Separation of parasporin-1-binding protein by two-dimensional electrophoresis. Left panel: control (SBED-labeled BSA). Right panel: SBED-labeled parasporin-1.
Fig. 4 shows effects of anti-beclin-1 antibody (upper panel), anti-TMOD-3 antibody (middle panel) and anti-cytochrome C antibody (lower panel) on the binding of parasporin-1 to HeLa cells.
Fig. 5 shows the localization of beclin-1 to the cell membrane. (A) Flow cytometric analysis of beclin-1 expression on HeLa cell surface. (B) Analysis of beclin-1 expression by Western blotting. Upper panel: whole cells, lower panel: membrane fractions containing organelle.
Fig. 6 shows cell death of HeLa cells induced by anti-beclin-1 antibody.

### Best Mode for Embodying the Invention

### 1. Method and agent for determining the sensitivity of cells to parasporin-1

The present invention provides a method of determining the sensitivity of cells to parasporin-1. The method of sensitivity determination of the present invention comprises measuring the expression level of beclin-1 polypeptide on the cell membrane or in a membrane fraction of a cell, and determining the sensitivity of the cell to parasporin-1 on the basis of a positive correlation between the expression level and sensitivity to parasporin-1. The method of sensitivity determination of the present invention is useful for, for example, distinguishing tumors on which an antitumor drug is effective and tumors on which the same is ineffective, in developing parasporin-1 as the antitumor drug.

Parasporin-1 is a publicly known protein that was isolated from the B. thuringiensis A1190 strain (Katayama, H. et al., J. Biochem. 137, 17-25 (2005)). Parasporin-1 is produced as an about 81-kDa precursor, and upon partial degradation by protease treatment, it forms a complex consisting of an about 56-kDa polypeptide and an about 15-kDa polypeptide. This complex possesses binding activity for beclin-1, and binds to beclin-1 expressed on a cell surface to induce cell death due to apoptosis to the cell. Herein, "parasporin-1" means this active complex unless otherwise stated.

"The sensitivity of a cell to parasporin-1" refers to the degree of inducibility of cell death (particularly apoptosis) when the cell is exposed to parasporin-1. "A cell is sensitive to parasporin-1" means that cell death (particularly apoptosis) is induced when the cell is exposed to parasporin-1.

Cells used in the method of sensitivity determination of the present invention are mammalian cells. Mammals include laboratory animals, including rodents such as mice, rats, hamsters and guinea pigs, and rabbits; domestic animals such as pigs, cattle, goat, horses, sheep, and minks; companion animals such as dogs and cats; primates such as humans, monkeys, rhesus monkeys, marmosets, orangutans, and chimpanzees, and the like. Cells used in the method of sensitivity determination of the present invention are preferably human cells.

Cells used in the method of sensitivity determination of the present invention include cultured cells, cells separated from living organisms, cells comprised in tissues separated from living organisms, cells in living organisms and the like. Cells used in the method of sensitivity determination of the present invention are tumor cells or non-tumor cells, preferably tumor cells.

Beclin-1 is a publicly known protein (Liang, H. X. et al., Nature 402, 672-676 (1999); Atia, V. M. et al., Genomics 59, 59-65 (1999); Qu, X. et al., J. Clin. Investig. 112, 1809-1820 (2003); Yue, Z. et al., Proc. Natl. Acad. Sci. U.S.A., b, 15077-15082 (2003); Pattingre, S. et al., Cell, 122, 927-939 (2005); Liang, X. H. et al., J. Virol. 72, 8586-8596 (1998); Furuya, D. et al., Exp. Cell Res. 307, 26-40 (2005)), and the amino acid sequence and mRNA (cDNA) sequence thereof are also publicly known. The beclin-1 that can be used in the present invention is the beclin-1 of one of the above-described mammals. As the amino acid sequence of human beclin-1, the amino acid sequence shown by SEQ ID NO:2 is known (NCBI Integrated Database Accession No. NP_003757, version NP_003757.1); as the nucleotide sequence of human beclin-1 mRNA (cDNA) that encodes the same, the nucleotide sequence shown by SEQ ID NO:1 is known (NCBI Integrated Database Accession No. NM_003766, version NM_003766.2/coding region: 134-1486). As orthologs thereof, mouse beclin-1 [amino acid sequence: SEQ ID NO:4 (NCBI Integrated Database Accession No. NP_062530, version NP_062530.2), mRNA(cDNA) nucleotide sequence: SEQ ID NO:3 (NCBI Integrated Database Accession No. NM_019584, version NM_019584.3/coding region: 139-1485)], rat beclin-1[amino acid sequence: SEQ ID NO:6 (NCBI Integrated Database Accession No. NP_446191, version NP_446191.1), mRNA (cDNA) nucleotide sequence: SEQ ID NO:5 (NCBI Integrated Database Accession No. NM_053739, version NM_053739.2/coding region: 225-1571)] and the like are known.

A measurement of the expression level of beclin-1 polypeptide on the cell membrane or in a membrane fraction is normally performed ex vivo.

The expression level of beclin-1 polypeptide can be measured by an immunochemical technique using an antibody that specifically recognizes beclin-1 polypeptide. Immunochemical techniques include flow cytometric analysis, radioimmunoassay (RIA method), ELISA (Methods in Enzymol. 70: 419-439 (1980)), Western blotting, immunohistological staining and the like.

Parasporin-1 binds to beclin-1 expressed on the cell surface and induces cell death to the cell. Therefore, the expression level of beclin-1 polypeptide on the cell membrane (that is, on the cell surface) or in a membrane fraction better correlates with the sensitivity of the cell to parasporin-1 than the expression level of beclin-1 polypeptide in the whole cell. Therefore, in the method of sensitivity determination of the present invention, the expression level of beclin-1 polypeptide on the cell membrane or in a membrane fraction, more preferably the expression level of beclin-1 polypeptide on the cell membrane, is measured. "A membrane fraction" includes the membranes constituting organellae and the cell membrane. A membrane fraction can be prepared from a cell by a method known per se such as centrifugation. In measuring the expression level of beclin-1 polypeptide on the cell membrane, immunochemical techniques such as flow cytometric analysis and immunohistological staining are suitably used. In measuring the expression level of beclin-1 polypeptide in a membrane fraction, immunochemical techniques such as radioimmunoassay (RIA method), ELISA (Methods in Enzymol. 70: 419-439 (1980)), and Western blotting are suitably used.

An antibody that specifically recognizes beclin-1 polypeptide can be produced by conventional method of antibody production using beclin-1 polypeptide or a partial peptide having the antigenicity thereof as an immunogen. As mentioned herein, antibodies include, but are not limited to, natural type antibodies such as polyclonal antibodies and monoclonal antibodies (mAbs), chimeric antibodies, humanized antibodies, single-stranded antibodies and human antibodies that can be produced using gene recombination technology, and binding fragments thereof. Preferably, the antibody is a polyclonal antibody, a monoclonal antibody or a binding fragment thereof. A binding fragment means a partial region of one of the above-described antibodies having the specific binding activity; specifically, for example, F(ab')₂, Fab', Fab, Fv, sFv, dsFv, sdAb and the like can be mentioned (Exp. Opin. Ther. Patents, Vol.6, No.5, p.441-456, 1996). The class of antibody is not particularly limited; antibodies of any itotypes such as IgG, IgM, IgA, IgD and IgE are encompassed. Preferably, the class is IgG or IgM, and in view of the ease of purification and the like, the class is more preferably IgG.

Next, on the basis of the measured expression level of beclin-1 polypeptide on the cell membrane or in a membrane fraction, the sensitivity of a cell to parasporin-1 is determined. As shown in an Example described below, as the expression level of beclin-1 polypeptide on the cell membrane or in a membrane fraction in a cell increases, the sensitivity of the cell to parasporin increases. The above-described determination is made on the basis of such a positive correlation between the expression level of beclin-1 polypeptide and sensitivity to parasporin-1.

In an embodiment, for example, if the cell expresses beclin-1 polypeptide on the cell membrane or in a membrane fraction, it can be determined that the cell is sensitive to parasporin-1. If the cell does not express beclin-1 polypeptide on the cell membrane or in a membrane fraction, it can be determined that the cell is non-sensitive to parasporin-1.

In another embodiment, for example, a cell that does not express beclin-1 polypeptide on the cell membrane or in a membrane fraction, and that is non-sensitive to parasporin-1 (negative control), and a cell that expresses beclin-1 polypeptide on the cell membrane or in a membrane fraction, and that is sensitive to parasporin-1 (positive control), are provided in advance, and the expression level of beclin-1 polypeptide on the cell membrane or in a membrane fraction in the measurement subject cell is compared with those of the positive control and negative control. Alternatively, a diagram of the correlation between the expression level of beclin-1 polypeptide on the cell membrane or in a membrane fraction in a cell and the sensitivity to parasporin-1 may be generated in advance, and the expression level of beclin-1 polypeptide on the cell membrane or in a membrane fraction in the measurement subject cell may be compared with the correlation diagram. This comparison of expression level is preferably made on the basis of the presence or absence of a significant difference.

Then, judging from the comparative results for beclin-1 polypeptide expression levels, if the beclin-1 polypeptide expression level on the cell membrane or in a membrane fraction in the measurement subject cell is relatively high, it can be determined that the sensitivity of the cell to parasporin-1 is relatively high. Conversely, if the beclin-1 polypeptide expression level on the cell membrane or in a membrane fraction in the measurement subject cell is relatively low, it can be determined that the sensitivity of the cell to parasporin-1 is relatively low.

The present invention also provides an agent for determining the sensitivity of a cell to parasporin-1, containing the above-described antibody that specifically recognizes beclin-1 polypeptide (referred to as the agent (I) of the present invention). The agent (I) of the present invention can be a kit for determining the sensitivity of a cell to parasporin-1. By using the agent (I) of the present invention, it is possible to easily determine the sensitivity of a cell to parasporin-1 by the above-described method.

An antibody that specifically recognizes beclin-1 polypeptide can be dissolved in water or an appropriate buffer solution (e.g., TE buffer, PBS and the like) to obtain an appropriate concentration, and preserved at about -20°C to 4°C.

The agent (I) of the present invention may further contain other ingredients necessary for embodying the method according to the method of measuring the expression level of beclin-1 polypeptide.

For example, the agent (I) of the present invention can further contain a labeled secondary antibody, color developing substrate, blocking liquid, washing buffer solution, ELISA plate, blotting membrane and the like.

### 2. Agent and method for inhibiting the cytotoxicity of parasporin-1

As stated above, parasporin-1 binds to beclin-1 polypeptide on the cell surface of a target cell to induce cell death (particularly apoptosis) to the cell. Therefore, by administering an effective amount of beclin-1 polypeptide or an antibody that specifically recognizes beclin-1 polypeptide, the binding of parasporin-1 to beclin-1 polypeptide on the cell surface is inhibited, and the cytotoxicity of parasporin-1 is inhibited. Therefore, the present invention provides an inhibitor of the cytotoxicity of parasporin-1 comprising beclin-1 polypeptide or an antibody that specifically recognizes beclin-1 polypeptide (the inhibitor of the present invention).

The beclin-1 polypeptide that can be used in the present invention is the beclin-1 polypeptide of one of the above-described mammals. As mammalian beclin-1 polypeptides, human beclin-1 polypeptides (for example, a polypeptide comprising the amino acid sequence shown by SEQ ID NO:2), mouse beclin-1 polypeptides (for example, a polypeptide comprising the amino acid sequence shown by SEQ ID NO:4), rat beclin-1 polypeptides (for example, a polypeptide comprising the amino acid sequence shown by SEQ ID NO:6) and the like can be mentioned. Beclin-1 polypeptide can be obtained by culturing a transformant incorporating a beclin-1 expression vector designed on the basis of the gene sequence thereof, and isolating/purifying the desired product from the culture using a commonly known means of purification such as column chromatography.

As an antibody that specifically recognizes beclin-1 polypeptide, an antibody that can be used in the above-described method of sensitivity determination of the present invention can be used; as the antibody, an antibody capable of inhibiting the specific binding of beclin-1 polypeptide and parasporin-1 polypeptide (neutralizing antibody) is preferable. The antibody used for the inhibitor of the present invention
is preferably an antibody that does not exhibit an activity to induce cell death to a beclin-1-expressing cell when brought into contact with the cell. The epitope recognized by the antibody used in the inhibitor of the present invention is not particularly limited, as far as the antibody inhibits the cytotoxicity of parasporin-1. As an excellent antibody that potently inhibits the cytotoxicity of parasporin-1 and little induces cell death to beclin-1-expressing cells, an antibody that recognizes a site of human beclin-1 polypeptide comprising threonine 72 (Thr72) as an epitope can be mentioned.

The inhibitor of the present invention may contain, in addition to the above-described beclin-1 polypeptide or antibody that specifically recognizes beclin-1 polypeptide, an optionally chosen carrier, for example, a pharmaceutically acceptable carrier.

As examples of the pharmaceutically acceptable carrier, excipients such as sucrose and starch; binders such as cellulose and methylcellulose; disintegrants such as starch and carboxymethylcellulose; lubricants such as magnesium stearate and Aerosil; flavoring agents such as citric acid and menthol; preservatives such as sodium benzoate and sodium hydrogen sulfite; stabilizers such as citric acid and sodium citrate; suspending agents such as methylcellulose and polyvinylpyrrolidone; dispersing agents such as surfactants; diluents such as water and physiological saline; base waxes; and the like can be mentioned, which, however, are not to be construed as limiting.

The content of beclin-1 polypeptide or an antibody that specifically recognizes beclin-1 polypeptide in the inhibitor of the present invention is, for example, about 0.01 to 100% by weight of the entire pharmaceutical composition.

In an embodiment, the inhibitor of the present invention is used in vivo. In this case, the inhibitor is orally or parenterally administered to a subject animal. As preparations suitable for oral administration, liquids, capsules, sachets, tablets, suspensions, emulsions and the like can be mentioned. Preparations suitable for parenteral administration (for example, subcutaneous injection, intramuscular injection, topical injection, intraperitoneal administration and the like) include aqueous and non-aqueous isotonic sterile injectable liquids, which may comprise an antioxidant, a buffer solution, a bacteriostatic agent, an isotonizing agent and the like. Aqueous and non-aqueous sterile suspensions can also be mentioned, which may comprise a suspending agent, a solubilizer, a thickening agent, a stabilizer, an antiseptic and the like. These preparations can be encapsulated in containers such as ampoules and vials for unit dosage or a plurality of dosages. It is also possible to freeze-dry the active ingredient and a pharmaceutically acceptable carrier, and store the preparation in a state that may be dissolved or suspended in an appropriate sterile vehicle just before use.

When the inhibitor of the present invention is used in vivo, the dosage of the inhibitor of the present invention varies depending on the activity and choice of the active ingredient, seriousness of illness, recipient animal species, the recipient's drug receptivity, body weight, age, and the like, and cannot be generalized; however, the dosage is normally about 0.001 to about 500 mg/kg, based on the amount of active ingredient, per day for an adult human.

The inhibitor of the present invention is safely administered to the above-described mammal in a way such that the active ingredient thereof will be delivered to a cell that is a target of parasporin-1 (a cell that expresses beclin-1 on cell surface).

In another embodiment, the inhibitor of the present invention is used in vitro. In this case, beclin-1 polypeptide or an antibody that specifically recognizes beclin-1 polypeptide is administered (added) to a medium comprising parasporin-1 and a target cell therefor (a cell that expresses beclin-1 on cell surface).

When the inhibitor of the present invention is used in vitro, the dosage of the inhibitor of the present invention varies depending on the activity and choice of the active ingredient and the like, and cannot be generalized; however, the dosage is normally about 0.1 to 500 µg/ml, based on the final concentration of active ingredient in the culture.

Because beclin-1 polypeptide or an antibody that specifically recognizes beclin-1 polypeptide is capable of inhibiting the binding of parasporin-1 to beclin-1 polypeptide on cell surface to inhibit the manifestation of cytotoxicity by parasporin-1, the inhibitor of the present invention is useful in preventing/treating cell death and poisoning that can be induced by parasporin-1. The inhibitor of the present invention is also useful in analyzing the mechanism for the manifestation of cytotoxicity by parasporin-1.

### 3. Method of screening for substances capable of inhibiting the cytotoxicity of parasporin-1

The present invention provides a method of screening for substances capable of inhibiting the cytotoxicity of parasporin-1, comprising the steps shown below (the screening method (I) of the present invention):
(I) evaluating the degree of binding of parasporin-1 to beclin-1 polypeptide in the presence of a test substance, and
(II) selecting a substance that has inhibited the binding of parasporin-1 to beclin-1 polypeptide as a substance capable of inhibiting the cytotoxicity of parasporin-1.

The step (I) can further comprise comparing the degree of binding of parasporin-1 to beclin-1 polypeptide in the presence of the test substance with the degree of binding of parasporin-1 to beclin-1 polypeptide in the absence of the test substance.

The test substance subjected to the screening method (I) of the present invention may be any commonly known compound or a novel compound; examples include nucleic acids, sugars, lipids, proteins, peptides, organic low molecular compounds, compound libraries prepared using combinatorial chemistry technology, random peptide libraries, or naturally occurring ingredients derived from microorganisms, animals, plants, marine organisms and the like, and the like.

As stated above, parasporin-1 is produced as an about 81- kDa precursor, and upon partial degradation by protease treatment, it forms an active (possessing cytotoxicity) complex consisting of an about 56-kDa polypeptide and an about 15-kDa polypeptide. In the screening method (I) of the present invention, this active complex is preferably used as parasporin-1. The parasporin-1 used in the screening method (I) of the present invention is preferably one that has been isolated and purified. "Isolated and purified" means having been treated to remove compounds other than the desired product.

In the screening method (I) of the present invention, a beclin-1 polypeptide that can be used in the above-described inhibitor of the present invention can be used as beclin-1 polypeptide. In an embodiment, the beclin-1 polypeptide used in the screening method (I) of the present invention has been isolated and purified. In another embodiment, beclin-1 polypeptide expressed on the cell surface of a cell naturally or forcibly expressing beclin-1 (preferably a cell of one of the above-described mammals) is used in the screening method (I) of the present invention. The cell naturally expressing beclin-1 is not particularly limited, as far as it potentially expresses beclin-1; as the cell, a primary culture cell, a cell line induced from the primary culture cell and the like can be used. Examples of cells naturally expressing beclin-1 include cervical carcinoma cells (HeLa cells and the like). Cells forcibly expressing beclin-1 include a transformant incorporating an expression vector capable of expressing beclin-1, and the like. In the vector, a polynucleotide that encodes beclin-1 polypeptide is integrated downstream of a promoter functional in the host cell in a way such that beclin-1 can be expressed in the host cell.

The evaluation in the step (I) is normally performed in an appropriate culture medium or buffer solution. The degree of binding of parasporin-1 to beclin-1 polypeptide can be evaluated by a method of interaction analysis well known in the art, for example, surface plasmon resonance, binding assay, immunological techniques and the like.

For example, parasporin-1 and beclin-1 polypeptide are brought into contact with each other in the presence of a test substance by immobilizing either parasporin-1 or beclin-1 polypeptide on a chip, and loading a solution containing the other polypeptide and the test substance on the chip. Next, the binding and dissociation of parasporin-1 to and from beclin-1 polypeptide are measured in the presence of the test substance by the surface plasmon resonance method, and are compared with the binding and dissociation obtained when a control solution without the test substance is loaded on the chip.

Alternatively, parasporin-1 and beclin-1 polypeptide are brought into contact with each other in the presence of a test substance by immobilizing either parasporin-1 or beclin-1 polypeptide on a plate, and adding a solution containing the other polypeptide labeled with an appropriate radioisotope or fluorescer and the test substance to the plate. Next, with the amount of labeling substance bound onto the plate as an indicator, the binding of parasporin-1 to beclin-1 polypeptide in the presence of the test substance is measured, and compared with the binding in the absence of the test substance.

Alternatively, parasporin-1 and beclin-1 polypeptide are brought into contact with each other in the presence of a test substance by adding a solution containing parasporin-1 labeled with an appropriate fluorescer and the test substance to a cell expressing beclin-1 polypeptide on the cell surface. Next, the binding of parasporin-1 to beclin-1 polypeptide on the cell surface in the presence of the test substance is measured by flow cytometry, and compared with the binding in the absence of the test substance.

Then, on the basis of the comparative results for binding and dissociation rates or the amount bound, a test substance that inhibits the binding of parasporin-1 and beclin-1 polypeptide is selected as a substance capable of inhibiting the cytotoxicity of parasporin-1.

Because a substance which can be obtained by the screening method (I) of the present invention is capable of inhibiting the manifestation of cytotoxicity by parasporin-1, it is useful as a candidate substance for the prevention/treatment of cell death and poisoning that can be induced by parasporin-1. A substance which can be obtained by the screening method (I) of the present invention is also useful in analyzing the mechanism for the manifestation of cytotoxicity by parasporin-1.

### 4. Combination comprising parasporin-1 and beclin-1 polypeptide

The present invention provides a combination comprising parasporin-1 and beclin-1 polypeptide (the combination of the present invention). The combination of the present invention is useful as a reagent for screening for a substance capable of inhibiting the cytotoxicity of parasporin-1.

In an embodiment, parasporin-1 and beclin-1 polypeptide are comprised in the combination of the present invention while in a separated state. In this embodiment, parasporin-1 and beclin-1 are housed in different containers, respectively, and the plurality of containers are housed in one package.

In another embodiment, parasporin-1 and beclin-1 polypeptide are comprised in the combination of the present invention while in a mixed state, to constitute a composition.

The parasporin-1 comprised in the combination of the present invention is the same as one that can be used in the screening method (I) of the present invention.

The beclin-1 polypeptide comprised in the combination of the present invention is the same as one that can be used in the screening method (I) of the present invention. The beclin-1 polypeptide comprised in the combination of the present invention is preferably an isolated and purified polypeptide.

Using the combination of the present invention, it is possible to easily perform the screening method (I) of the present invention.

### 5. Agent and method for inducing cell death using an antibody that specifically recognizes beclin-1 polypeptide

As stated in an Example described below, when an effective amount of an antibody that specifically recognizes beclin-1 polypeptide is administered, the antibody binds to beclin-1 on the cell surface of a target cell, resulting in the induction of cell death (particularly apoptosis) to the cell as with the use of parasporin-1. Therefore, by administering an effective amount of beclin-1 polypeptide or an antibody that specifically recognizes beclin-1 polypeptide, the binding of parasporin-1 to beclin-1 polypeptide on the cell surface is inhibited, whereby the cytotoxicity of parasporin-1 is inhibited.

A targeted cell for the agent (II) of the present invention is a cell expressing beclin-1 polypeptide on the cell surface. Targeted cells for the agent (II) of the present invention include cultured cells, cells separated from living organisms, cells comprised in tissues separated from living organisms, cells in living organisms and the like. The cell is a tumor cell or non-tumor cell, preferably a tumor cell.

As an antibody that specifically recognizes beclin-1 polypeptide, that can be used in the agent (II) of the present invention, the same as one that can be used in the above-described agent (I) of the present invention can be mentioned. The antibody is preferably an antibody that exhibits an activity to induce cell death to a beclin-1-expressing cell when coming into contact with the cell. The epitope recognized by the antibody used in the agent (II) of the present invention is not particularly limited, as far as the antibody possesses an activity to induce cell death. Because a polyclonal antibody capable of recognizing whole beclin-1 polypeptide is potently active to induce cell death, it is preferably used in the agent (II) of the present invention. Such a polyclonal antibody can be acquired by administering a polypeptide comprising the full-length amino acid sequence of beclin-1 polypeptide, along with a commercially available adjuvant (for example, Freund complete or incomplete adjuvant), to an animal subcutaneously or intraperitoneally about 2 to 4 times at intervals of 2 to 3 weeks (the antibody titer of partially drawn serum should be measured by a publicly known antigen-antibody reaction, and an elevation thereof should be confirmed in advance), collecting whole blood about 3 to 10 days after final immunization, and purifying the antiserum. Animals to which the antigen is to be administered include non-human mammals such as rats, mice, rabbits, goat, guinea pigs, and hamsters.

The agent (II) of the present invention may contain, in addition to the above-described antibody that specifically recognizes beclin-1 polypeptide, an optionally chosen carrier, for example, a pharmaceutically acceptable carrier.

As examples of the pharmaceutically acceptable carrier, excipients such as sucrose and starch; binders such as cellulose and methylcellulose; disintegrants such as starch and carboxymethylcellulose; lubricants such as magnesium stearate and Aerosil; flavoring agents such as citric acid and menthol; preservatives such as sodium benzoate and sodium hydrogen sulfite; stabilizers such as citric acid and sodium citrate; suspending agents such as methylcellulose and polyvinylpyrrolidone; dispersing agents such as surfactants; diluents such as water and physiological saline; base waxes; and the like can be mentioned, which, however, are not to be construed as limiting.

The content of an antibody that specifically recognizes beclin-1 polypeptide in the agent (II) of the present invention is, for example, about 0.01 to 100% by weight of the entire pharmaceutical composition.

In an embodiment, the agent (II) of the present invention is used in vivo. In this case, the inhibitor is orally or parenterally administered to a subject animal. As preparations suitable for oral administration, liquids, capsules, sachets, tablets, suspensions, emulsions and the like can be mentioned. Preparations suitable for parenteral administration (for example, subcutaneous injection, intramuscular injection, topical injection, intraperitoneal administration and the like) include aqueous and non-aqueous isotonic sterile injectable liquids, which may contain an antioxidant, a buffer solution, a bacteriostatic agent, an isotonizing agent and the like. Aqueous and non-aqueous sterile suspensions can also be mentioned, which may contain a suspending agent, a solubilizer, a thickening agent, a stabilizer, an antiseptic and the like. These preparations can be encapsulated in containers such as ampoules and vials for unit dosage or a plurality of dosages. It is also possible to freeze-dry the active ingredient and a pharmaceutically acceptable carrier, and store the preparation in a state that may be dissolved or suspended in an appropriate sterile vehicle just before use.

When the agent (II) of the present invention is used in vivo, the dosage of the agent (II) of the present invention varies depending on the activity and choice of the active ingredient, seriousness of illness, recipient animal species, the recipient's drug receptivity, body weight, age, and the like, and cannot be generalized; however, the dosage is normally about 0.001 to about 500 mg/kg, based on the amount of active ingredient, per day for an adult human.

The agent (II) of the present invention is safely administered to the above-described mammal in a way such that the active ingredient thereof will be delivered to a targeted cell (a cell expressing beclin-1 on the cell surface).

In another embodiment, the agent (II) of the present invention is used in vitro. In this case, an antibody that specifically recognizes beclin-1 polypeptide is administered (added) to a medium containing a targeted cell (a cell that expresses beclin-1 on the cell surface).

When the agent (II) of the present invention is used in vitro, the dosage of the agent (II) of the present invention varies depending on the activity and choice of the active ingredient and the like, and cannot be generalized; however, the dosage is normally about 0.1 to 500 µg/ml, based on the final concentration of active ingredient in the culture.

The agent (II) of the present invention is useful as an antitumor agent in preventing and treating a tumor (preferably a tumor expressing beclin-1 polypeptide on the cell surface). The tumor is exemplified by, but not limited to, cervical carcinoma, gastric cancer, lung cancer, colorectal cancer, brain tumor, ovarian cancer, prostatic cancer, liver cancer, malignant lymphoma and the like.

### 6. Screening method for substances capable of inducing cell death

As stated above, an antibody that specifically recognizes beclin-1 polypeptide or parasporin-1 binds to beclin-1 polypeptide expressed on the cell surface to induce cell death (apoptosis) to the cell. Therefore, any substance, like parasporin-1, capable of binding to beclin-1 polypeptide is capable of inducing cell death to cells that express beclin-1 on the cell surface. Therefore, the present invention provides a method of screening for a substance capable of inducing cell death (apoptosis), comprising the steps shown below (the screening method (II) of the present invention):
(I) evaluating the degree of binding of a test substance to beclin-1 polypeptide, and
(II) selecting a test substance that has bound to beclin-1 polypeptide as a substance capable of inducing cell death.

The step (I) can further comprise comparing the degree of binding of the test substance to beclin-1 polypeptide with the degree of binding of the test substance to a polypeptide other than beclin-1 (control polypeptide).

The test substance subjected to the screening method (II) of the present invention, as in the screening method (I), may be any commonly known compound or a novel compound.

In the screening method (II) of the present invention, the same beclin-1 polypeptide as one used in the screening method (I) can be used. In the screening method (II) of the present invention, as in the screening method (I), an isolated and purified beclin-1 polypeptide or beclin-1 polypeptide expressed on the cell surface of a cell naturally or forcibly expressing beclin-1 is used as beclin-1 polypeptide.

The evaluation in the step (I) is normally performed in an appropriate culture medium or buffer solution. The degree of binding of a test substance to beclin-1 polypeptide can be evaluated, as in the screening method (I), by a method of interaction analysis well known in the art, for example, surface plasmon resonance, binding assay, immunological techniques and the like.

For example, a test substance and beclin-1 polypeptide are brought into contact with each other by immobilizing beclin-1 polypeptide on a chip, and loading a solution containing the test substance onto the chip. Next, the binding and dissociation of the test substance to or from beclin-1 polypeptide are measured by the surface plasmon resonance method, and compared with the binding and dissociation of the test substance to or from the control peptide when the control peptide is immobilized on the chip.

Alternatively, a test substance and beclin-1 polypeptide are brought into contact with each other by immobilizing beclin-1 polypeptide on a plate, and adding a solution containing the test substance labeled with an appropriate radioisotope or fluorescer to the plate. Next, with the amount of labeling substance bound onto the plate as an indicator, the binding of the test substance to beclin-1 polypeptide is measured, and compared with the binding to the control polypeptide.

Alternatively, a test substance and beclin-1 polypeptide are brought into contact with each other by adding a solution containing the test substance labeled with an appropriate fluorescer to a cell expressing beclin-1 polypeptide on the cell surface. Next, the binding of the test substance to beclin-1 polypeptide on the cell surface is measured by flow cytometry, and compared with the binding of the test substance to a control cell not expressing beclin-1.

Then, on the basis of the comparative results for binding and dissociation rates or the amount bound, a test substance that has bound to beclin-1 polypeptide is selected as a substance (candidate substance) capable of inducing cell death.

The screening method (II) of the present invention may comprise after the step (II) a step for bringing a substance selected through the step (II) into contact with a cell expressing beclin-1 on the cell surface, and selecting a substance that has induced cell death (preferably apoptosis) to the cell as a substance confirmed to induce cell death. Contact of the test substance and the cell expressing beclin-1 on the cell surface is performed in a culture medium. A culture medium is chosen as appropriate according to the kind of cell; examples include a minimal essential medium (MEM), Dulbecco's Modified Eagle's Medium (DMEM) and the like containing about 5 to 20% fetal calf serum. Culturing conditions are also determined as appropriate; for example, the pH of the medium is about 6 to about 8, culturing temperature is normally about 30 to about 40°C, and culturing time is about 12 to about 72 hours. Cell death induction can be evaluated by the TUNNEL method, DNA ladder detection and the like.

Substances obtained by the screening method (II) of the present invention are useful as candidate substances for the development of new antitumor agents.

The present invention is explained in more detail in the following by referring to Examples and the like, which are not to be construed as limitative.

### Examples

### 1. Binding of parasporin-1 to HeLa cell

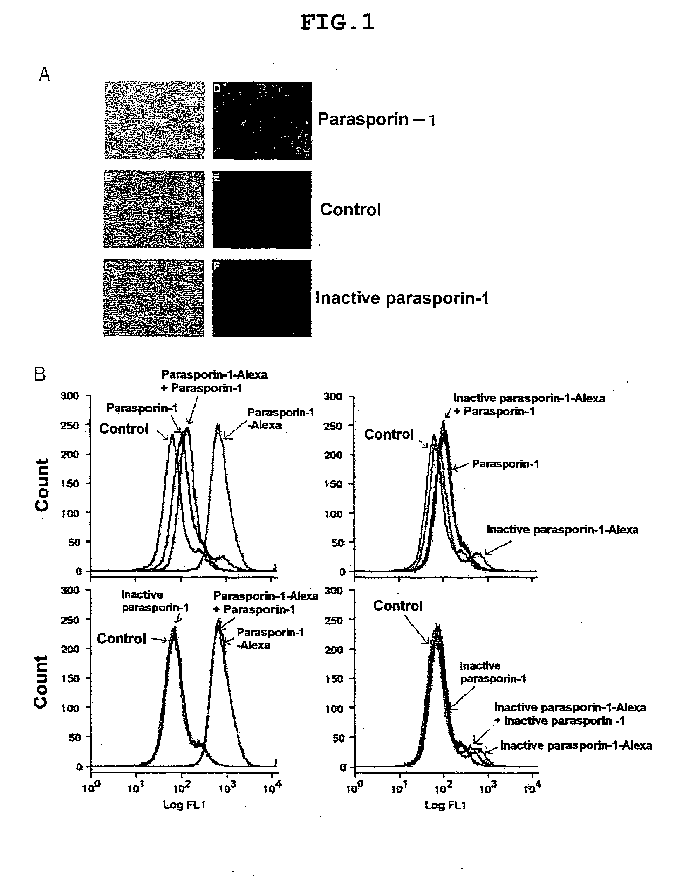

To confirm that parasporin-1 can bind to the target cell surface, HeLa cells were treated with parasporin-1 labeled with Alexa Fluor 488 dye, and immunostained with an anti-Alexa Fluor 488 dye antibody. The cells treated with active paraspotin-1 could be stained with a fluorescence antibody, but staining could not be detected in control and the cells treated with inactive parasporin-1 treated at 100°C for 5 min (Fig. 1A). HeLa cells were treated with parasporin-1 labeled with Alexa Fluor 488 dye, and the binding of parasporin-1 to the cells was analyzed by flow cytometry (Fig. 1B). The cells treated with active labeled parasporin-1 showed increased fluorescence, and the increased fluorescence disappeared by adding non-labeled parasporin-1 in much excess (40-fold of labeled parasporin-1) (Fig. 1B, upper left panel).

### 2. Comparison of binding to cell lines with various parasporin-1 sensitivity

Parasporin-1 is a cytotoxin with high cell specificity.

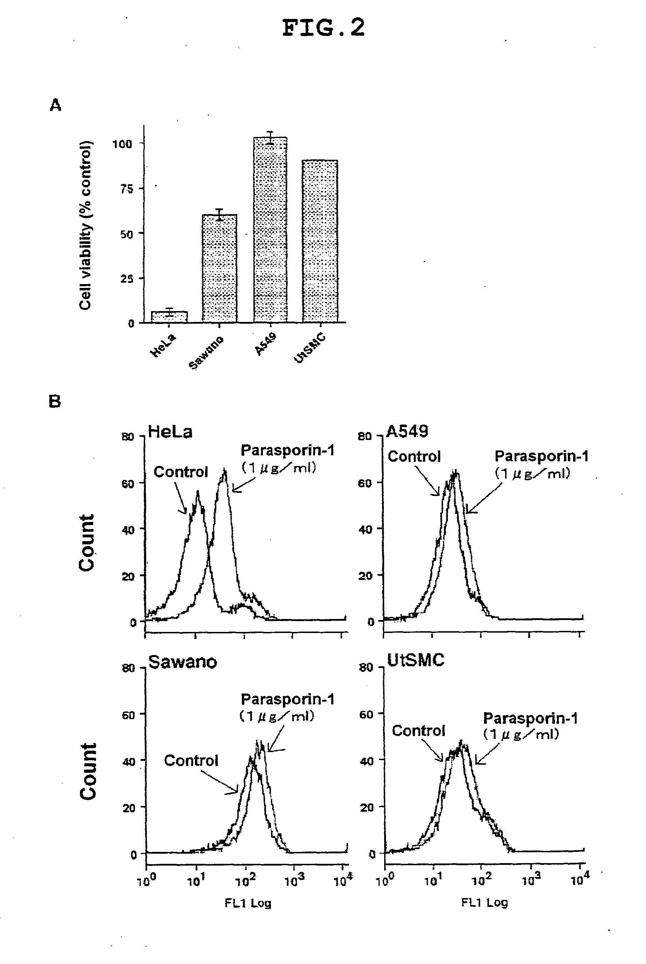

Fig. 2A shows sensitivity of HeLa, A549, Sawano and normal uterus smooth muscle cells (UtSMC) to parasporin-1 (1 µg/ml). To show correlation between parasporin-1 sensitivity of cell and binding of parasporin-1 to cell membrane, the binding property of parasporin-1 to various cells was analyzed by flow cytometry. HeLa cell showed parasporin-1 dependent peak shift but other cell lines hardly showed a peak shift.

### 3. Screening and identification of parasporin-1 receptor using photoreactive chemical crosslinking agent

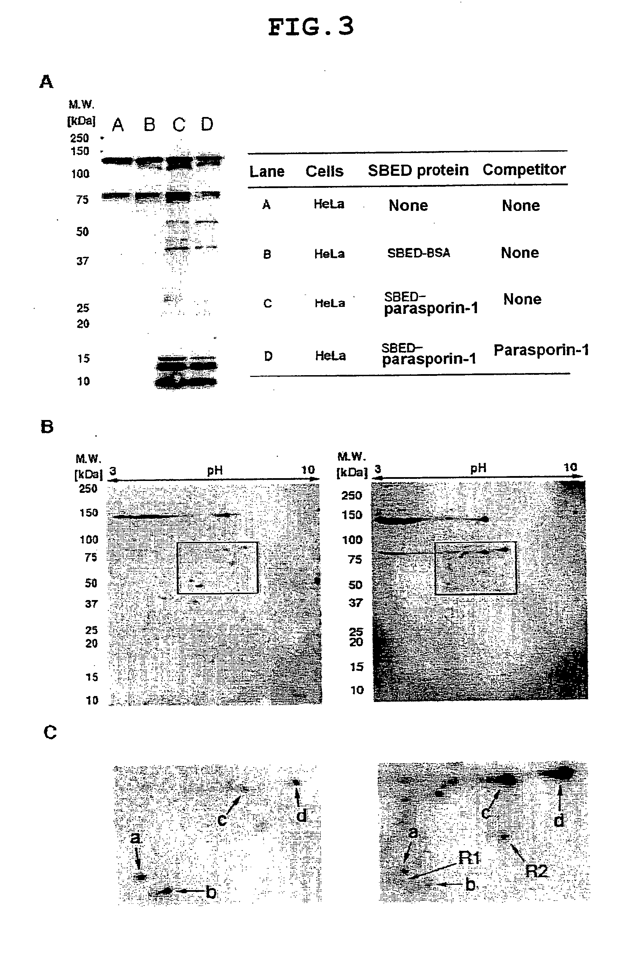

Parasporin-1 receptor was screened for using parasporin-1 labeled with a photoreactive chemical crosslinking agent (SBED). As a result of chemical crosslinking, since biotin transfer occurs in a protein that has interacted with parasporin-1, a protein of HeLa cell to which biotin was transferred was screened for by Western blotting. As a control, SBED-labeled BSA was used. As a result of Western blot, proteins to which biotin was transferred in a parasporin-1 dependent manner were observed (Fig. 3A, lane C). It was futher confirmed that biotin translocation clearly disappeared by the coexistence of non-labeled parasporin-1 in a part of proteins in which biotin transfer had been observed (Fig. 3A, lane D). Although the protein was separated by one-dimensional electrophoresis and identification of a protein showing biotin translocation was tried, the identification failed since separation of protein was insufficient. Thus, the protein of the cell was further separated by two-dimensional electrophoresis, and a protein showing induction of biotin translocation was screened for. HeLa cells were treated with SBED-labeled BSA or SBED-labeled parasporin-1, and a crosslinking reaction was performed. The cell membrane was recovered from the treated cells, and a protein was separated by two-dimensional electrophoresis. For detection of a Biotintransferred protein, Western blotting using avidin-HRP was performed (Fig. 3B). As a result of two-dimensional electrophoresis, protein spots (R1, R2) were observed in pH 6 to pH 8, molecular weight 37 - 80 kDa, where parasporin-1 dependent biotin translocation was induced (Fig. 3C, right panel). R1 and R2 were prepared by two-dimensional electrophoresis. The prepared protein was identified by mass finger printing method. As a result, R1 and R2 were tropomodulin 3 (TMOD-3) and beclin-1, respectively.

### 4. Inhibitory effect of antibody on cell binding of parasporin-1

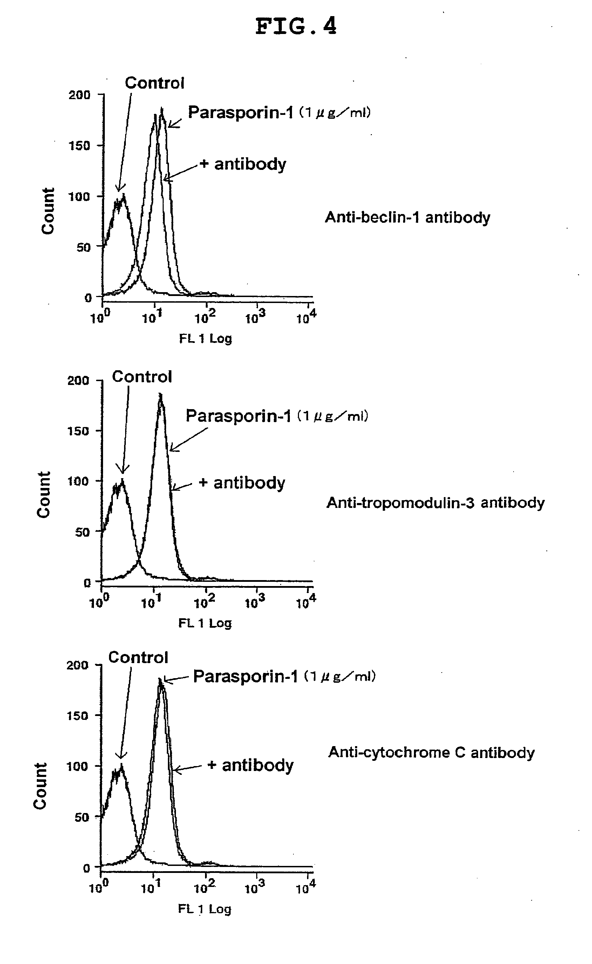

To confirm that parasporin-1 receptor candidate protein identified by mass finger printing method has a parasporin-1 receptor function, inhibition of cell membrane binding of parasporin-1 by antibodies against each candidate protein was examined. HeLa cells were treated in advance with anti-beclin-1 antibody, anti-cytochrome C antibody or anti-TMOD-3 antibody, and binding of fluorescence-labeled parasporin-1 to HeLa cells was detected by flow cytometry. As anti-beclin-1 antibody, a polyclonal antibody manufactured by Cell Signaling Technology was used. The polyclonal antibody was prepared by immunizing a rabbit with a synthesized partial peptide (KLH-conjugated) corresponding to the residue adjacent to threonine 72 of human beclin-1. The binding of parasporin-1 to HeLa cell was inhibited by anti-beclin-1 antibody (Fig. 4A), but it was not observed in anti-TMOD-3 antibody and anti-cytochrome C antibody (Fig. 4B and C). In addition, anti-beclin-1 antibody that inhibits binding of parasporin-1 to HeLa cell could also partially inhibit cytotoxicity of parasporin-1 against HeLa cell.

### 5. Localization of beclin-1 in cell membrane

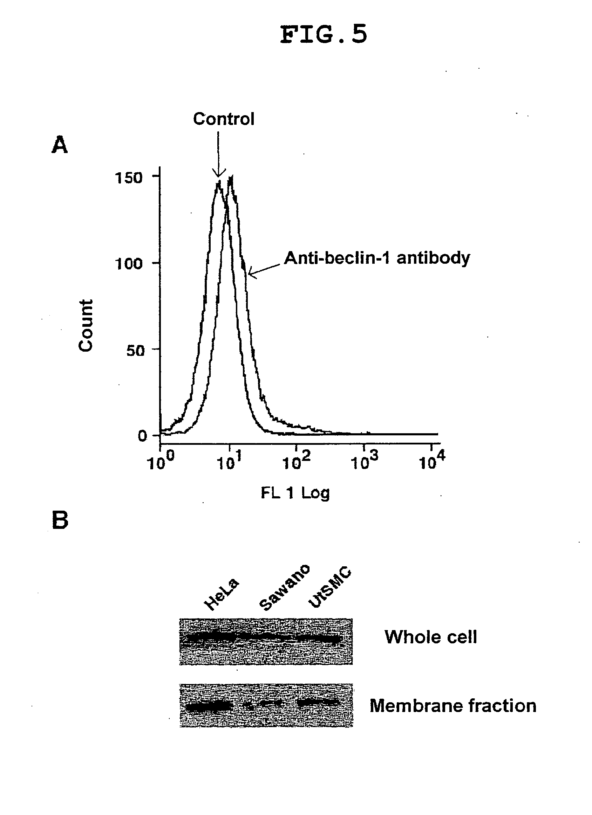

To show that beclin-1 is present on the cell membrane, HeLa cells were treated with anti-beclin-1 antibody (manufactured by Cell Signaling Technology), and the anti-beclin-1 antibody on the HeLa cell surface was detected with a fluorescence antibody by flow cytometry. As a result, fluorescence peak shift was observed in an anti-beclin-1 antibody dependent manner, suggesting that beclin-1 is present on the cell surface of HeLa cells (Fig. 5A). Then, the cellular components were fractionated by ultracentrifugation, and whether the membrane fractions including organelle contain beclin-1 was examined. Beclin-1 was detected by Western blotting. The experiment was performed not only with HeLa cells but also with other cell lines (Sawano, UtSMC) with different parasporin-1 sensitivity. The content of beclin-1 present in the cells was not much different between the cell lines (Fig. 5B, upper panel). However, the content of beclin-1 present in membrane fractions in Sawano and UtSMC was low as compared to HeLa cells (Fig. 5B, lower panel).

### 6. Cytotoxicity of anti-beclin-1 antibody

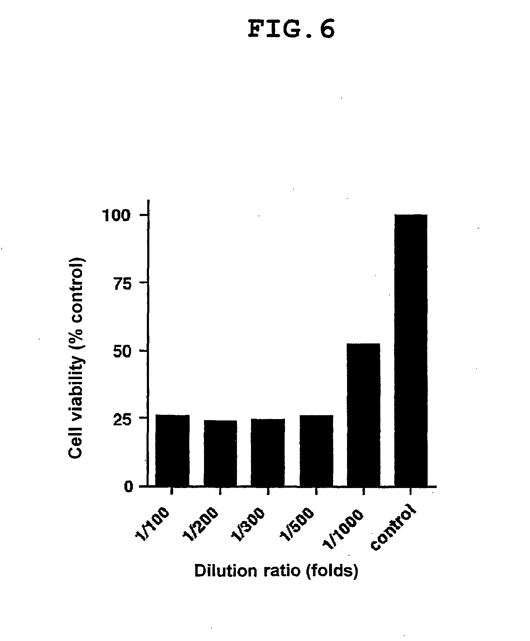

Cell death is induced by binding of parasporin-1 to beclin-1. Therefore, whether anti-beclin-1 antibody having higher affinity than parasporin-1 shows a cytotoxicity induction effect similar to that of parasporin-1 was examined. The anti-beclin-1 antibody used for this experiment was a polyclonal antibody manufactured by Affinity Bioreagents. The polyclonal antibody was prepared by immunizing a rabbit with a human beclin-1 protein (whole protein). The anti-beclin-1 antibody was diluted and HeLa cells were treated with the antibody at 37°C for 20 h. The survival rate of the cell was calculated by a color development method using an MTT reagent. The cell survival rate did not decrease in the control (preimmunized rabbit serum), whereas the cell survival rate remarkably decreased with the anti-beclin-1 antibody (Fig. 6). However, cell death was scarcely induced by the above-mentioned anti-peptide antibody (manufactured by Cell Signaling Technology) against beclin-1 having a limited binding region.

### Discussion

Experimental results suggesting the presence of a proteinous receptor of parasporin-1 on the HeLa cell membrane have been obtained heretofore. In this experiment, the receptor of cytotoxin parasporin-1 was identified to be beclin-1 by using a photoreactive chemical crosslinking agent.

The binding ability of parasporin-1 to the cell membrane surface of cell lines having various sensitivities to parasporin-1 showed high correlation with parasporin-1 sensitivity. Therefrom it was assumed that parasporin-1 sensitivity of a cell line is determined by the binding of parasporin-1 to the cell surface, namely, the amount or the presence or absence of a receptor. The amount of beclin-1 in the membrane fraction containing organelle of highly sensitive HeLa cell was high and that in the cells (Sawano and UtSMC) having low sensitivity was small. However, the content of beclin-1 in the whole cells of HeLa, Sawano and UtSMC was almost the same. These results suggest that the selectivity of cells to parasporin-1 toxicity depends on expression of beclin-1 on the cell surface. In addition, it is shown that a parasporin-1 treatment induces an increase in the intracellular calcium concentration of parasporin-1 sensitive cells, which triggers cytotoxicity (Katayama, H. et al., J. Biol. Chem. 282, 7742-7752 (2007)). This suggests that beclin-1 that functions as a extracellularly exposed receptor of parasporin-1 regulates the mechanism of calcium influx.

Beclin-1 has been identified as a protein relating to autophagy, and acts as a cancer suppressive factor. In addition, beclin-1 has been reported to be an intracellular protein that interacts with Bcl-2 and Bcl-X_{L}, which are the factors regulating apoptosis. However, it is not known that beclin-1 appears on the cell surface, and functions as a receptor. Therefore, it can be said that parasporin-1 has indicated new and unknown function of beclin-1. While the physiological meaning of beclin-1 present on the cell surface is completely unknown at the present stage, beclin-1 is assumed to be at least involved in the cellular phenomena other than autophagy and apoptosis induction.

Parasporin-1 is a toxic protein showing selective toxicity to the cell, and localization of its receptor, beclin-1, on the cell membrane surface was found in the parasporin-1 sensitive cells. Moreover, a treatment of HeLa cells (parasporin-1 high sensitive cell) with an anti-beclin-1 antibody resulted in the apoptosis induction via caspase-8, like that of parasporin-1. The foregoing suggests that cell membrane localization of beclin-1, which is a parasporin-1 receptor, can be used as one marker for distinguishing the kind of cells, and that the development of a new antitumor agent targeting extracellularly exposed beclin-1 is possible.

### Industrial Applicability

The present invention provides various uses of beclin-1 as a receptor of parasporin-1.
Using the method of the present invention for determining sensitivity to parasporin-1, it is possible to objectively determine the sensitivity of various cells to parasporin-1 on the basis of the expression level of parasporin-1 receptor definitely supported by molecular biologically.
Using the inhibitor of the cytotoxicity of parasporin-1 of the present invention, it is possible to potently suppress the cell death (apoptosis) induced by parasporin-1.
Using the cell death inducer of the present invention, it is possible to induce cell death (apoptosis) specifically to cells expressing beclin-1, and the inducer is useful as a cell-specific antitumor agent.
This application is based on a Japanese patent application No. 2008-090190 (filing date: March 31, 2008, the contents of which are incorporated in full herein.

## Claims

1. A method of determining the sensitivity of a cell to parasporin-1, comprising measuring the expression level of beclin-1 polypeptide on the cell membrane or in a membrane fraction of a cell, and determining the sensitivity of the cell to parasporin-1 on the basis of a positive correlation between the expression level of beclin-1 polypeptide and the sensitivity to parasporin-1.

2. The method according to claim 1, wherein the measurement of the expression level of beclin-1 polypeptide is performed using an antibody that specifically recognizes beclin-1 polypeptide.

3. An agent for determining the sensitivity of a cell to parasporin-1, comprising an antibody that specifically recognizes beclin-1 polypeptide.

4. An inhibitor of the cytotoxicity of parasporin-1, comprising beclin-1 polypeptide or an antibody that specifically recognizes beclin-1 polypeptide.

5. A method of inhibiting the cytotoxicity of parasporin-1, comprising administering beclin-1 polypeptide or an antibody that specifically recognizes beclin-1 polypeptide.

6. A method of screening for a substance capable of inhibiting the cytotoxicity of parasporin-1, comprising the steps shown below:
(I) evaluating the degree of binding of parasporin-1 to beclin-1 polypeptide in the presence of a test substance, and
(II) selecting a substance that has inhibited the binding of parasporin-1 to beclin-1 polypeptide as a substance capable of inhibiting the cytotoxicity of parasporin-1.

7. A combination comprising parasporin-1 and beclin-1 polypeptide.

8. A cell death inducer comprising an antibody that specifically recognizes beclin-1 polypeptide.

9. An antitumor agent comprising an antibody that specifically recognizes beclin-1 polypeptide.

10. A method of inducing cell death, comprising bringing an antibody that specifically recognizes beclin-1 polypeptide into contact with a cell.

11. The method according to claim 10, wherein the cell is a tumor cell.

12. A method of screening for a substance capable of inducing cell death, comprising the steps shown below:
(I) evaluating the degree of binding of a test substance to beclin-1 polypeptide, and
(II) selecting a test substance that has bound to beclin-1 polypeptide as a substance capable of inducing cell death.

13. The method according to claim 12, wherein the beclin-1 polypeptide used in (I) is beclin-1 polypeptide expressed on the cell membrane.

14. An antibody that specifically recognizes beclin-1 polypeptide, for use in inducing cell death.

15. An antibody that specifically recognizes beclin-1 polypeptide, for use in preventing or treating a tumor.

16. A method of inducing cell death in a mammal, comprising administering to the mammal an effective amount of an antibody that specifically recognizes beclin-1 polypeptide.

17. A method of preventing or treating a tumor in a mammal, comprising administering to the mammal an effective amount of an antibody that specifically recognizes beclin-1 polypeptide.
